# EUROPEAN PATENT APPLICATION

(11) **EP 0 823 481 A1**
(43) Date of publication of application: **11.02.1998**
(21) Application number: 96401764.4
(22) Date of filing: 09.08.1996
(51) Int. Cl.: C12N 15/82, C12N 15/29, A01H 5/00, A01N 65/00, C12Q 1/68, C07K 14/415

(54) **Resistance against nematodes**

(71) Applicant: KEYGENE N.V., NL-6700 AE Wageningen (NL)
(72) Inventor: Vos, Pieter, 3927 BD Renswoude (NL); Zabeau, Marc, 9000 Gent (BE); Simons, Guus, 6715 JL Ede (NL); Wijnbrandi, Jelle, 6702 AB Wageningen (NL)
(74) Representative: Desaix, Anne

(57) **Abstract**

The invention relates to genes capable of confering resistance against nematodes. Preferred nucleic acids of the invention are DNA sequences which are at least part of the DNA sequence provided on the figure or homologous thereto.

The invention further relates to genetically transformed plants which are resistant to nematodes.

## Description

### FIELD OF THE INVENTION

The present invention relates to resistance genes, DNA constructs, micro-organisms, plant cells and plants comprising said resistance genes. Furthermore the invention relates to genetically transformed plants which are resistant against nematodes. In addition, the invention relates to probes, and primers for the identification of the resistance genes and diagnostic kits comprising said probes and/or primers. Finally, the invention relates to polypeptides encoded by said resistance genes and the use of said polypeptides.

### BACKGROUND OF THE INVENTION

Plant pathogens are responsible for substantially losses of plants and plant products due to infection of the plant. Plant diseases, as a result of infection by plant pathogens or pests, cause damage to the plants and/or plant products, reduce production and yield, limit the kind of plants that can grow in certain geographic areas and as a result cause severe (financial) losses to the grower.

Plant parasitic nematodes occur worldwide and most of them live most of their life in the topsoil layer. Although losses caused by direct feeding of nematodes on plant roots is considered to be of minor importance, several species, among them the root-knot nematodes belonging to the *Meloidogyne* species, the cyst nematodes belonging to the *Heterodera* species and *Globodera* species and other nematodes such the *Nacobbus* species, cause severe damage and economic crop losses. Root-knot nematodes also occur throughout the world but are found more frequently and in greater numbers in areas with warmer climates and in greenhouses. The most important *Meloidogyne* species are *M. incognita*, *M. arenaria*, *M. hapla* and *M. javanic*a, of which *M. hapla* also occurs in more temperate climatic zones.

Different means for control of the plant pathogens exist, such as mechanical cultivation of the soil, chemical treatment with pesticides, including nematicides and insecticides, or crop rotation. However, for certain plant pathogens, especially nematodes, these means of control are insufficient to protect the plants from infection and resulting diseases. The only effective means of control involves plant host resistance (Russell, 1978, Plant Breeding for pest and disease resistance, Butterworths edit., 485 pp). The development of cultivars resistant to common plant pathogens is one of the major goals of plant breeders today, in order to reduce or ultimately eliminate the extensive need for pesticides. The burden for the environment of the large amounts of pesticides injected into the soil or sprayed on crops, trees etc. worldwide each year becomes too severe. Moreover, governmental regulations in Western countries restrict the use or even forbid the use of certain pesticides. Therefore, the need for plants which are resistant to one or more of their pathogens, or which have a reduced susceptibility to their attackers becomes more and more pressing. The development of resistant plants is one of the important objectives of current plant breeding programs. Plant genotypes susceptible for particular pathogens are crossed with resistant plant genotypes in order to introduce the resistant phenotype into the breeding line.

Damage by root-knot nematodes results primarily from the invasion of the plant roots by larvae which in a compatible relationship with the plant develop into a reproducing female. After invasion the larvae cause root cells to develop into giant cells on which they feed. Upon infection galls or knots are formed on the roots and the plant roots become otherwise disturbed, thickened and stunted. The root system thus disfunctions in the uptake of water and nutritional elements which damages the plant growth and development. Frequently damage to infected plants is increased by parasitic fungi attacking the weakened root tissue. Infected plants show reduced growth and smaller pale coloured leaves, with dwarf poor quality fruits or even without fruits, and tend to wilt in warmer climates (Agrios, 1988 in: Plant Pathology, Academic Press, Inc.). The damage and/or yield reduction caused by root-knot nematodes is substantial on the total agricultural production worldwide. In individual stand yield losses can be as high as 25-50 %, or even a crop may be killed.

In greenhouses root-knot nematodes can be controlled with steam sterilization of the soil or soil fumigation with nematicides. Under field conditions control can be achieved by the use of nematicides. However, the use of such, in some cases very persistent, chemicals is increasingly debated and in some countries the use of certain nematicides is even forbidden.

Breeding genetically resistant genotypes is the most reliable and effective way of controlling root-knot disease. For a number of crop species the availability of resistance within the related germplasm has been reported, e.g. potato, cotton, tobacco, wheat, soybean, tomato, eggplant, common bean and alfalfa. Resistance breeding is hampered by firstly the limited occurrence of (known) resistance genes in the available germplasm, secondly, in some plant species the existence of crossing barriers between the cultivated crop species and the resistance bearing related species, and thirdly, screening tests for resistance versus susceptibility to nematodes are laborious and often not reliable. Therefore, resistance breeding is very difficult or not to achieve, or if possible time consuming.

Successful introduction of resistance genes has been realized in tomato. The resistance gene *Mi* (*Meloidogyne incognita*) has been introduced into cultivated tomato, *Lycopersicon esculentum*, after crossing with the related wild species *L. peruvianum* (PI 128657), using embryo culture. The *Mi* gene confers resistance to various *Meloidoygne* spp. (Fassuliotis, 1991, in: Genetic Improvement of Tomato, Springer Verlag edit.). The *Mi* resistance gene is reported to be a monogenic dominant gene (Gilbert and McGuire, 1956, Proc. Am. Soc. Hortic. Sci. **68**, 437-442) and is located on tomato chromosome 6. It is also postulated that the introgressed region comprising the *Mi* locus is involved in conferring resistance to potato aphid (*Macrosiphum euphorbia*) (Kaloshian *et al*, 1995, Proc. Natl. Acad. Sci. USA, **92**, 622-625).

Plants have developed a complex defense mechanism against attack and infection by pathogens. To date, the exact mechanism of their defense system is not yet elucidated.

Nematode resistance in tomato is expressed after penetration. After the juvenile larva enters the root and establishes itself at a feeding site, a hypersensitive reaction (HR) adjacent to the head of the nematode is triggered that results in local death of the host cells. The nematode is also adversely affected by this HR and dies (Fassuliotis, 1991, in: Genetic Improvement of Tomato, Springer Verlag edit.). Wether or not there exists a gene-for-gene relationship sensu Flor (1956, Adv.Gen. **8**, 29-54) as is frequently the case in other plant-pathogen relationships where resistance is based on HR-incompatibility is unknown.

The isolation of plant genes without knowing their gene products is very laborious and difficult, because of the enormous genome sizes of plant species: e.g. tomato has a genome size of 1000 Mb (10⁹ base pairs of nuclear DNA), maize has a genome size of 3000 Mb and wheat has even more than 16 x 10⁹ base pairs. Searching for a specific gene among these billions of base pairs is only feasible when (i) there are enough molecular markers tightly linked to the gene of interest and (ii) there is good genetic material available (Tanksley *et al*., 1995, Trends in Genetics, **11**, p. 63-68).

Although, the isolation of a few resistance genes has been reported, none of these resistance genes are able to confer the host plant resistant to nematodes. Examples of such isolated resistance genes are: *RPS2* from Arabidopsis (resistance to *Pseudomonas syringae* expressing avrRpt2), *N* from tobacco (resistance to tobacco mosaic virus), *Cf-9* from tomato (resistance to the leaf fungal pathogen *Cladosporium fulvum* carrying *avr9*) and *L*^{*6*} from flax (resistance to the corresponding leaf rust fungal race) (Dangl, 1995, Cell **80**, 363-366).

The present invention provides the first isolated nematode resistance gene.

### SUMMARY OF THE INVENTION

The present invention relates to a nucleic acid comprising the *Mi* resistance gene which when present and expressed in a plant is capable of conferring said plant resistant to nematodes. Furthermore, the invention relates to the *Mi* resistance gene of which the DNA sequence is disclosed herein. The invention also relates to a gene product encoded by the *Mi* resistance gene. In addition, the present invention relates to DNA constructs, cosmids, vectors, bacterial strains, yeast cells and plant cells comprising the *Mi* resistance gene. In another aspect, the present invention relates to a genetically transformed plant, which is resistant to a nematode, said nematode being capable of infecting the untransformed plant. Furthermore, the invention relates to resistance genes which are homologous to the *Mi* resistance gene, and which, when present in a plant, are able of conferring said plant resistant to infection by pathogens.

Finally, the invention relates to oligonucleotides corresponding to the sequence of the *Mi* resistance gene or part thereof, and detection kits comprising said oligonucleotides.

### DESCRIPTION OF THE FIGURES

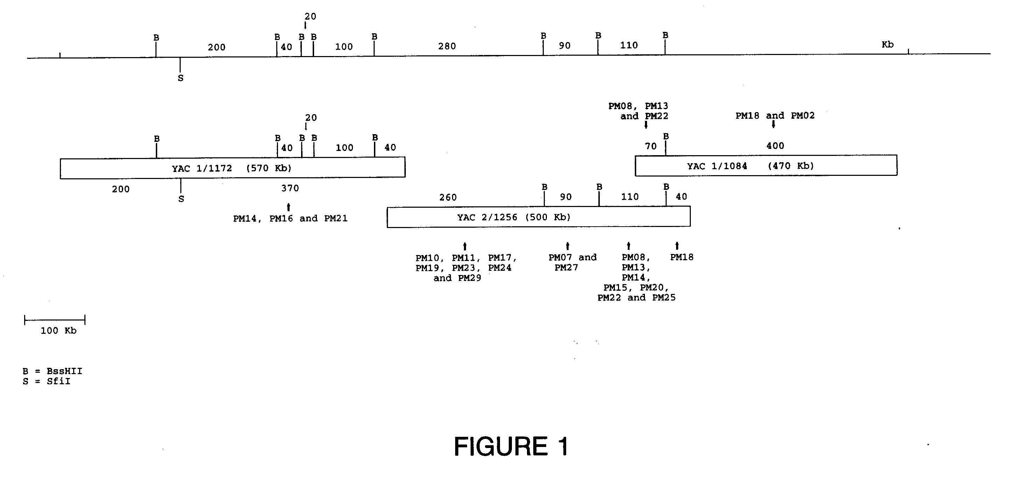

Figure 1 shows a physical map of YAC 1/1172, YAC 2/1256 and YAC 1/1084, with a size of 570, 500 and 470 kb respectively. The position of the *Sfi*I and *Bss*HII restriction sites and the size of the restriction fragments are indicated. The location of the various AFLP markers on the restriction fragments are indicated.

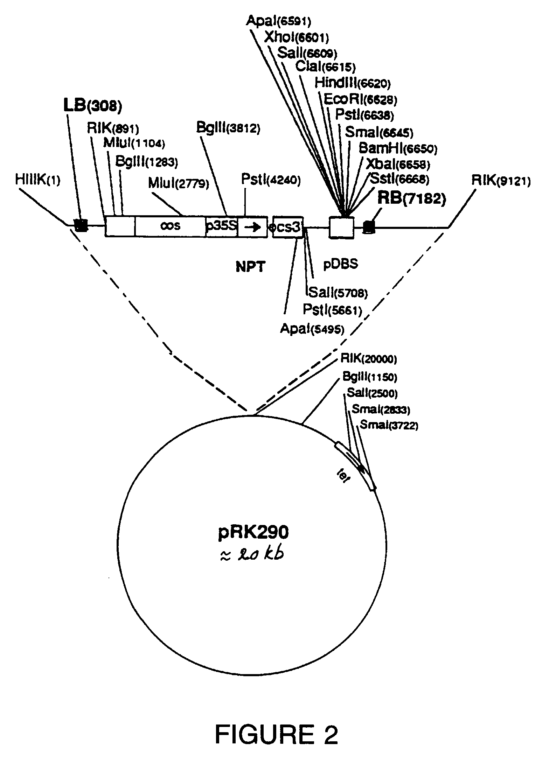

Figure 2 shows a schematic drawing of the binary cosmid vector pJJ04541 which is used to construct a cosmid library of YAC 1/546. Plasmid pRK290 (20 kb large) (Ditta *et al*, 1980, Proc. Natl. Acad. Sci. USA, **77**, 7347-7351) was used as starting vector. "Tet" refers to the gene conferring resistance to tetracyclin. "LB" signifies T-DNA left border repeat sequence, and "RB" signifies the right border repeat. The cauliflower mosaic virus 35S promoter sequence is indicated by "p35S", and "ocs3'" indicates the octopine synthase 3' end. "NPT" indicates neomycin phosphotransferase, and "cos" refers to the bacteriophage lambda cos site enabling *in vitro* packaging. "pDBS" indicates the polylinker of pBluescript (Stratagene, La Jolla, CA, USA).

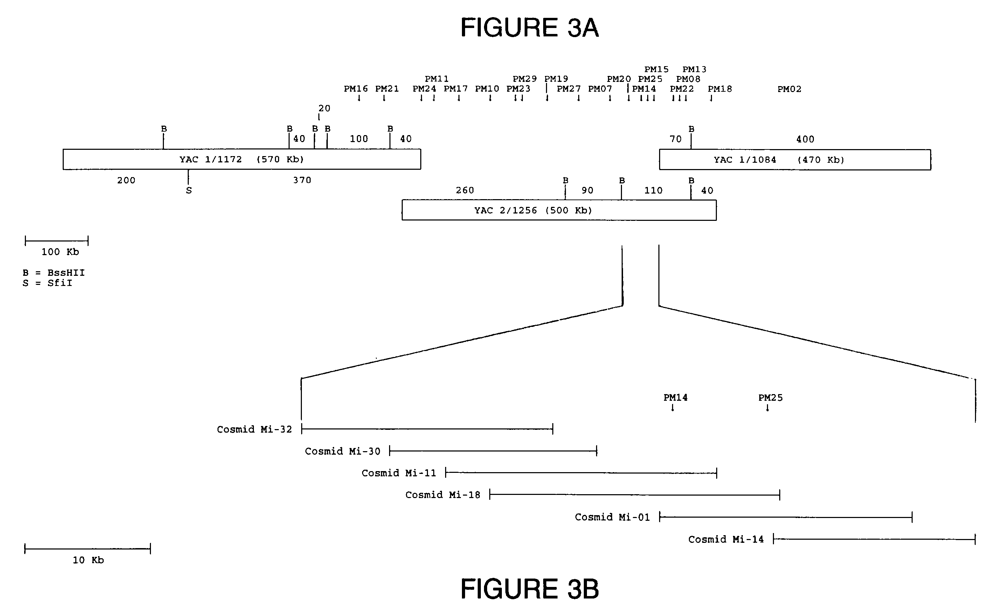

Figure 3A shows a schematic representation of the detailed position of the AFLP markers on YAC 1/1172, YAC 2/1256 and YAC 1/1084. Positioning is based on the cosmid contig constructed for the various defined regions.

Figure 3B shows a schematic representation of the cosmid contig of the region comprising the *Mi* resistance gene. The cosmids Mi-32, Mi-30, Mi-11, Mi-18, Mi-01 and Mi-14 are represented by horizontal lines. The location of the AFLP markers PM14 and PM25 is indicated.

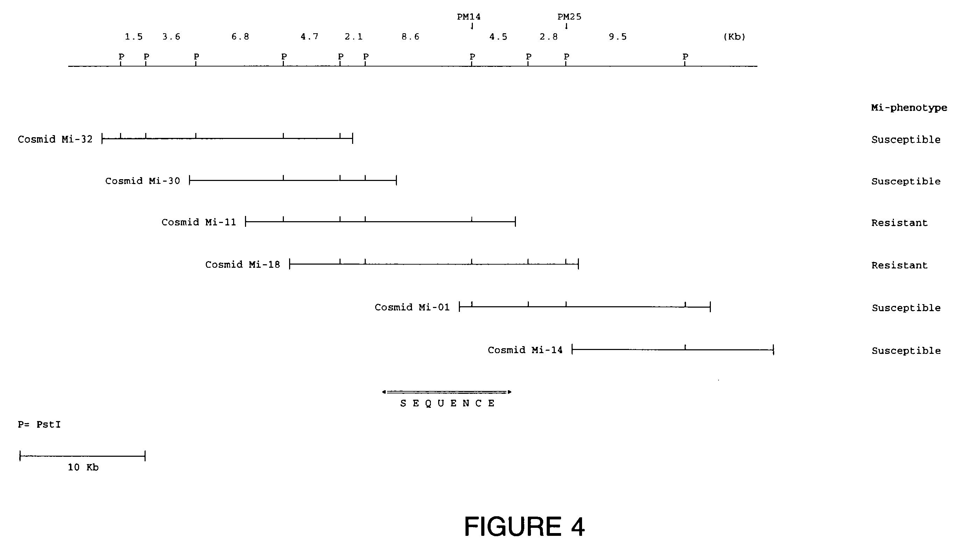

Figure 4 shows a physical fine map of the cosmids Mi-32, Mi-30, Mi-11, Mi-18, Mi-01 and Mi-14 for the restriction enzyme *Pst*I. The size of the *Pst*I fragments is indicated (in kb). The *Mi* phenotype of the R₀ plants comprising the various cosmids is indicated in the right end part of the figure. The DNA segment of which the nucleotide sequence was determined is indicated by a double line with a bidirectional arrow.

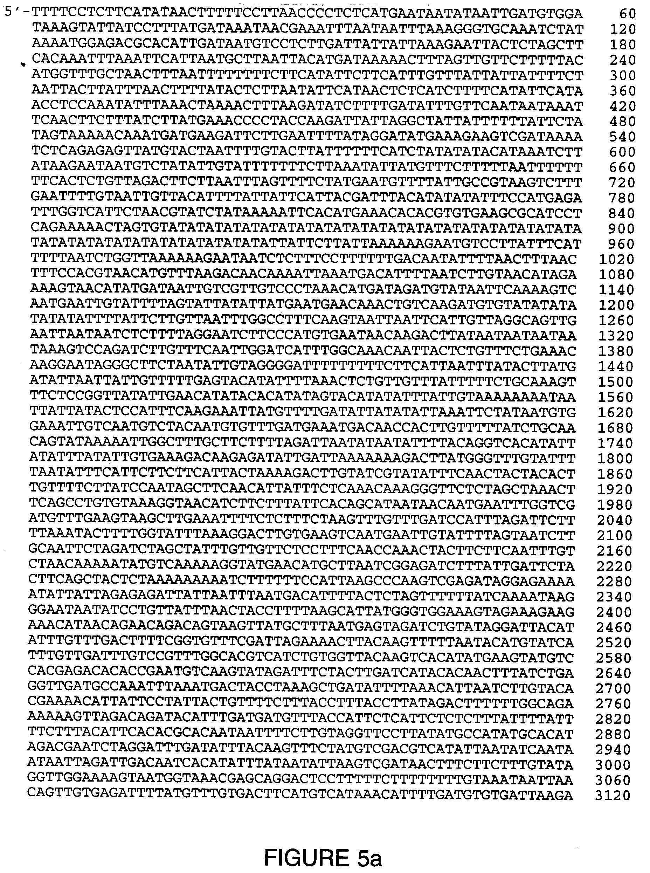

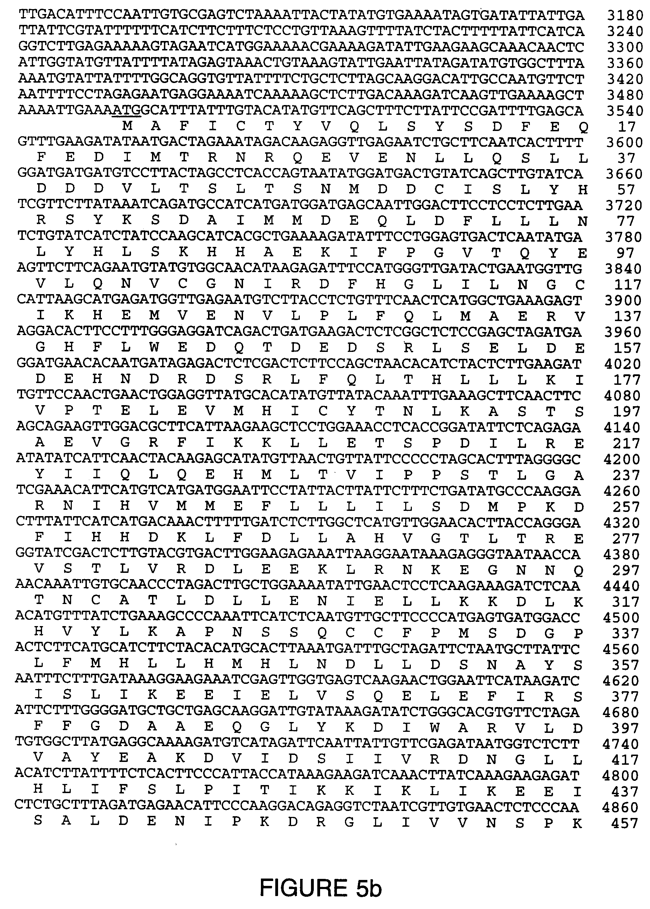

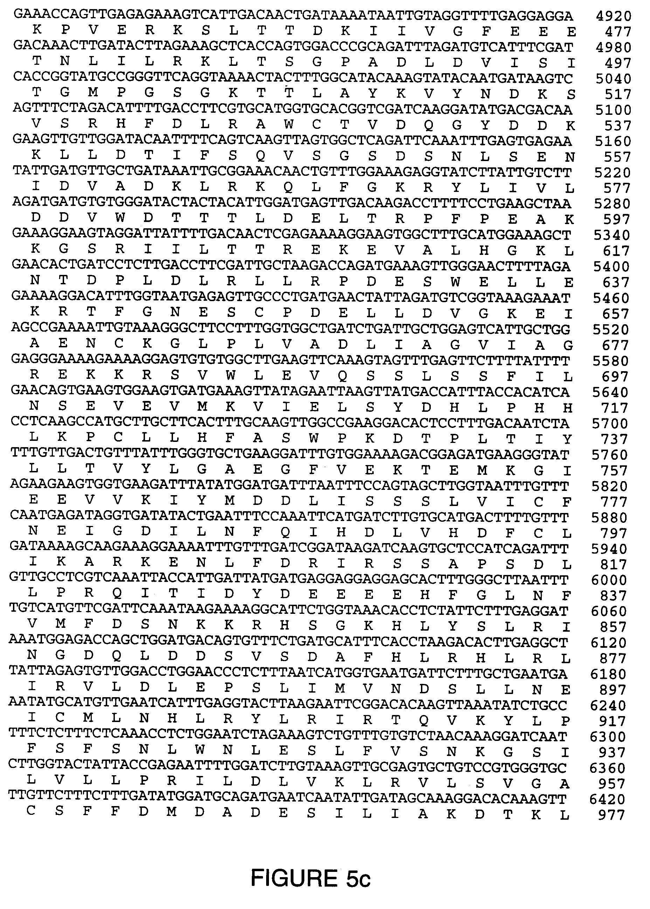

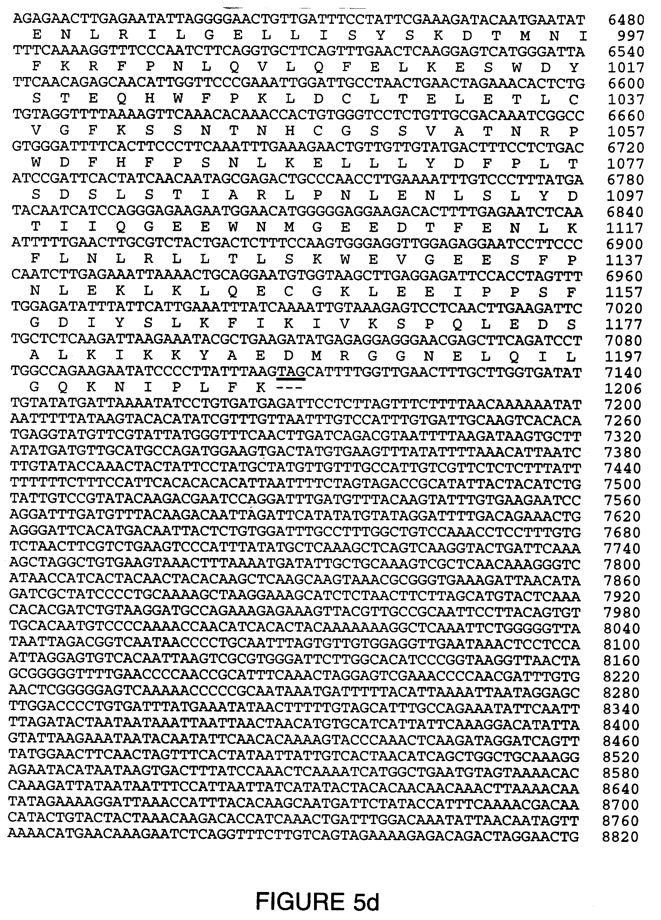

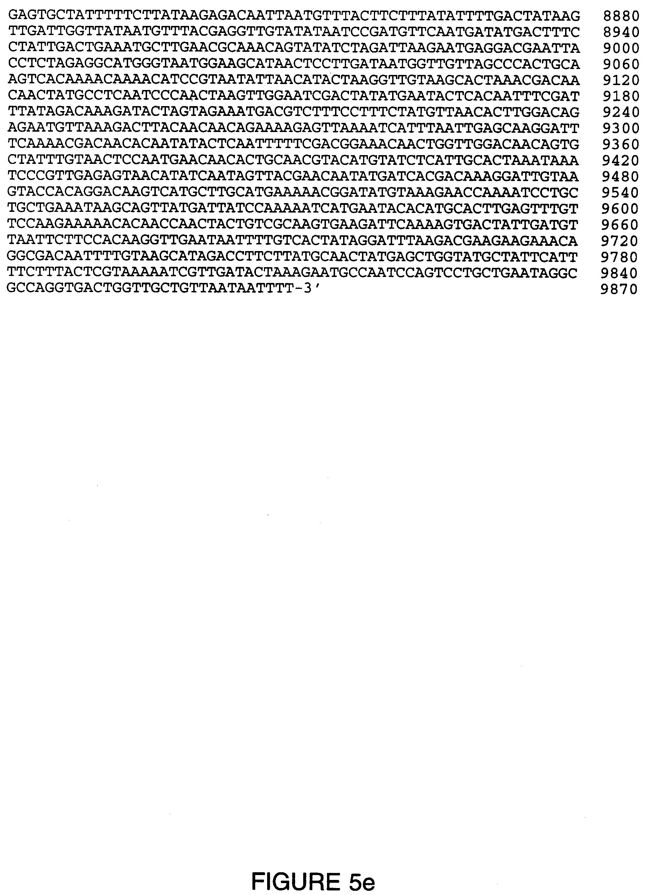

Figure 5 shows the nucleotide sequence of a DNA segment of approximately 9.9 kb around the AFLP marker PM14, and the deduced amino acid sequence of the *Mi* resistance gene. The initiation codon (ATG position 3491-3493) is underlined and the termination codon (TAG position 7109-7111) is double underlined.

The position of the AFLP marker PM14 is from nucleotide position 6921 (5'-TGCAG**GA**-3') to nucleotide position 7034 (5'-**AGA**TTA-3').

### DETAILED DESCRIPTION OF THE INVENTION

In the description and examples that follow, a number of terms are used herein. In order to provide a clear and consistent understanding of the specification and claims, including the scope to be given such terms, the following definitions are provided.
- nucleic acid: a double-stranded DNA molecule;
- oligonucleotide: a short single-stranded DNA molecule;
- primers: in general, the term primer refers to a single-stranded DNA molecule which can prime the synthesis of DNA;
- nucleic acid hybridization: a method for detecting related DNA sequences by hybridization of single-stranded DNA on supports such as nylon membrane or nitrocellulose filter papers. Nucleic acid molecules that have complementary base sequences will reform the double-stranded structure if mixed in solution under the proper conditions. The double-stranded structure will be formed between two complementary single-stranded nucleic acids even if one is immobilized on a support. In a Southern hybridization procedure, the latter situation occurs;
- hybridization probe: to detect a particular DNA sequence in the Southern hybridization procedure, a labelled DNA molecule or hybridization probe is reacted to the fractionated DNA bound to a support such as nylon membrane or nitrocellulose filter paper. The areas on the filter that carry DNA sequences complementary to the labelled DNA probe become labelled themselves as a consequence of the reannealing reaction. The areas of the filter that exhibit such labelling can then be detected according to the type of label used. The hybridization probe is generally produced by molecular cloning of a specific DNA sequence or by synthesizing a synthetic oligonucleotide;
- homologous sequence: a sequence which has at least 50 %, preferably 60 %, more preferably 70 %, most preferably 80 % or even 90 % sequence identity with the particular sequence, whereby the length of sequences to be compared for nucleic acids is generally at least 120 nucleotides, preferably 200 nucleotides and more preferably 300 nucleotides and the length of sequences to be compared for polypeptides is generally at least 40 amino acid residues, preferably 65 amino acid residues and more preferably 100 amino acid residues. Alternatively, a homologous sequence refers to a sequence which can hybridize under stringent conditions to a particular sequence, and/or a DNA sequence coding for a polypeptide which has substantially the same properties as the polypeptide encoded by the particular DNA sequence, and/or a DNA sequence coding for a polypeptide having the same amino acid sequence as the polypeptide encoded by the particular DNA sequence and/or an amino acid sequence in which some amino acid residues have been changed with respect to the amino acid sequence of the particular polypeptide without substantially effecting the major properties of said polypeptide;
- stringent conditions refer to hybridization conditions which allow a nucleic acid sequence to hybridize to a particular sequence. In general, high stringent conditions refer to the hybridization conditions which allow a nucleic acid sequence of at least 50 nucleotides and preferably about 200 or more nucleotides to hybridize to a particular sequence at about 65 °C in a solution comprising about 1 M salt, preferably 6 x SSC or any other solution having a comparable ionic strength, and washing at 65 °C in a solution comprising about 0,1 M salt, or less, preferably 0,2 x SSC or any other solution having a comparable ionic strength. These conditions allow the detection of sequences having about 90 % or more sequence identity. In general, lower stringent conditions refer to the hybridization conditions which allow a nucleic acid sequence of at least 50 nucleotides and preferably about 200 or more nucleotides to hybridize to a particular sequence at about 45 °C in a solution comprising about 1 M salt, preferably 6 x SSC or any other solution having a comparable ionic strength, and washing at room temperature in a solution comprising about 1 M salt, preferably 6 x SSC or any other solution having a comparable ionic strength. These conditions allow the detection of sequences having up to 50 % sequence identity. The person skilled in the art will be able to modify these hybridization conditions in order to identify sequences varying in identity between 50 % and 90 %;
- promoter: a transcription regulation region upstream from the coding sequence containing the regulatory sequences required for the transcription of the adjacent coding sequence and includes the 5' non-translated region or so called leader sequence of mRNA;
- terminator: a region downstream of the coding sequence which directs the termination of the transcription, also called the 3' non-translated region, which includes the poly-adenylation signal;
- resistance gene: a nucleic acid comprising a coding sequence as depicted in Figure 5, or part thereof, or any corresponding or homologous sequence;
- nematode(s): *Meloidogyne* spp. such as *Meloiodogyne incognita*, *M*. *arenaria* or *M. javanica*, or any other genotype which is not able to infect a host having a resistance gene according to the invention, such as but not limited to other root-knot nematodes, such as *M. hapla*, cyst nematodes such as *Heterodera* spp. or *Globodera* spp., or other nematodes such as *Nacobbus* spp., insects, such as potato aphid or any other plant pathogen or pest;
- resistance gene product: a polypeptide having an amino acid sequence as depicted in Figure 5, or part thereof, or any homologous amino acid sequence;
- R₀ plant: primary regenerant from a transformation experiment, also denoted as transformed plant or transgenic plant;

In the present invention we have been able to identify and isolate the *Meloidogyne incognita* (*Mi*) resistance gene. The gene was cloned from a tomato genotype which is resistant to *Meloidogyne incognita*. The isolated *Mi* resistance gene according to the invention can be transferred to a susceptible host plant using Agrobacterium mediated transformation or any other known transformation method, and is involved in conferring the host plant resistant to plant pathogens, especially to nematodes. The host plant can be tomato or any other genotype that is infected by said plant pathogen.

The present invention provides also a nucleic acid sequence comprising the *Mi* resistance gene, which is depicted in Figure 5.

With the *Mi* resistance gene according to the invention, one has an effective means of control against plant pathogens and/or pests, since the gene can be used for transforming susceptible plant genotypes thereby producing genetically transformed plants having a reduced susceptibility or being preferably resistant to a plant pathogen or pest. In particular, a plant which is genetically transformed with the *Mi* resistance gene according to the invention has a reduced susceptibility to root-knot nematodes.

In a preferred embodiment the *Mi* resistance gene comprises the coding sequence provided in Figure 5 or any corresponding or homologous sequence, preceded by a promoter region and followed by a terminator region. The promoter region should be functional in plant cells and, preferably, corresponds to the native promoter region of the *Mi* resistance gene. However, it should be recognized that any heterologous promoter region can be used in conjunction with the coding sequences, as long as it is functional in plant cells. Preferably, a constitutive promoter is used, such as the CaMV 35 S promoter or T-DNA promoters, all well known to those skilled in the art. Furthermore, a suitable terminator region should be functional in plant cells all well known to those skilled in the art.

In addition the invention relates to the *Mi* resistance gene product which is encoded by the *Mi* resistance gene according to the invention and which has an amino acid sequence provided in Figure 5, or which is homologous to the deduced amino acid sequence or part thereof. Furthermore, the *Mi* resistance gene product can be used for raising antibodies against it, which antibodies can be used for the detection of the presence of the *Mi* resistance gene product.

In another aspect of the invention, the *Mi* resistance gene can be used for the design of oligonucleotides which are complementary to one strand of the DNA sequence as described in Figure 5, or part thereof, which can be used as hybridization probes, being accordingly labelled to allow detection, for the screening of genomic DNA or cDNA libraries for homologous genes. Homologous sequences which can hybridize to the probe under stringent hybridization conditions, and which encode for a gene product that is involved in conferring reduced susceptibility or resistance to a plant against a plant pathogen which normally infects said plant, are comprised within the scope of the present invention.

In another aspect of the invention oligonucleotides are designed based on the *Mi* resistance gene sequence, such that they can be used as hybridization probes in Southern analysis. These probes can be used as molecular markers to distinguish plant genotypes having the resistance gene and plant genotypes lacking the resistance gene. Such a probe can be used as an additional tool in selection. In a preferred embodiment of the invention, oligonucleotides are designed based on the *Mi* resistance gene sequence, such that they can be used as primers in an amplification reaction, such as polymerase chain reaction (PCR), whereby the formation of an amplification product indicates the presence of the *Mi* resistance gene in a certain plant genotype. In a particular embodiment of the invention said primers direct the amplification of polymorphic fragments, so called molecular markers, which are closely linked to the *Mi* resistance gene. In a preferred embodiment said primers are used in selective restriction fragment amplification to identify AFLP markers, which are closely linked to the *Mi* resistance gene. The invention also relates to diagnostic kits, comprising oligonucleotides according to the invention, for the detection of the presence or absence of the *Mi* resistance gene within a genotype under study. Such a diagnostic kit circumvents the use of a laborious disease assay to screen for genotypes having the resistance gene or not.

Furthermore the invention relates to DNA constructs comprising a DNA sequence corresponding to the coding sequence of the *Mi* resistance gene and regulatory sequences functional in plant cells. Said regulatory sequences are either homologous or heterologous to the coding sequences of the *Mi* resistance gene. Preferably, said DNA construct comprises a nucleic acid whose sequence is provided in Figure 5, or part thereof.

The invention relates also to DNA constructs comprising the regulatory sequences, and more preferably the promoter region of the *Mi* resistance gene in conjunction with a structural gene sequence heterologous to said regulatory sequences.

The invention relates also to a DNA vector comprising a DNA construct according to the invention. Suitable vectors can be cloning vectors, transformation vectors, expression vectors, etc...., which are well known to those skilled in the art.

Furthermore, cells harbouring a vector comprising a DNA sequence corresponding to the sequence as described in Figure 5 or part thereof, or homologous thereto, are within the scope of the invention. Moreover, cells carrying a DNA construct according to the invention, are within the scope of this invention.

In one preferred embodiment of the invention, a genetically transformed plant is obtained by introducing the *Mi* resistance gene within the genome of said plant, having a susceptible genotype to nematodes, using standard transformation techniques, wherein said genetically transformed plant is resistant to nematodes.

In another embodiment of the invention, the *Mi* resistance gene can be transferred, using generally known transformation techniques, to a heterologous systems, such as but not limited to melon, tobacco, *Arabidopsis thaliana*, potato, sugarbeet, rapeseed, cucumber, pepper, eggplant. A heterologous system refers to a plant species which is different from the plant species from which the resistance gene was isolated.

The DNA sequence comprising the *Mi* resistance gene as provided in the present invention has numerous applications of which some are described herein but which are not limiting the scope of the invention.

The present invention will be further described in detail in view of the isolation of the *Mi* resistance gene present in tomato lines which are resistant to root-knot nematodes. For the isolation of the *Mi* resistance gene we have used a map-based cloning (positional cloning) strategy, comprising the following steps:
(1) identification of molecular markers linked to the *Mi* resistance gene,
(2) construction of a high molecular weight genomic YAC library,
(3) physical mapping of the molecular markers on the YAC clones and YAC contig building,
(4) construction of a cosmid library of the YAC clones harbouring the linked molecular markers,
(5) physical fine mapping and cosmid contig building,
(6) genetic characterization of tomato mutants susceptible to root-knot nematodes,
(7) transformation of susceptible plants with the cosmids forming the contig,
(8) complementation analysis.

For the identification of molecular markers, we have used the selective restriction fragment amplification technology, hereinafter also denoted as AFLP™ technology, which randomly amplifies a subset of DNA fragments out of a complex mixture of many DNA fragments and said amplified fragments generate fingerprints that can be analyzed. In general, total DNA of different genotypes of the same plant species are subjected to the AFLP technology and the different AFLP fingerprints obtained from the different genotypes are compared. Fragments that are present in one genotype and absent in another genotype are polymorphic fragments and are denoted as AFLP markers. The selectivity in AFLP reactions is obtained by using randomly chosen selective nucleotides at the 3' end of the PCR primers immediately adjacent to the nucleotides of the restriction enzyme site. In an AFLP screening the DNA to be studied is subjected to different primer combinations. The total amount of different primers that can be used is determined by the number of selective nucleotides that are added to the 3' end (4 primers with 1 selective nucleotides, 16 primers with 2 selective nucleotides, 64 primers with 3 selective nucleotides). If two different restriction enzymes are used than there are twice the amount of primers. Those primers can be used in different combination. If all possible combinations are used in an AFLP screening, than all the fragments present should have been amplified with one of the primer combinations (Zabeau and Vos, EP 0534858).

For the identification of AFLP markers linked to the *Mi* resistance gene different tomato lines were subjected to an AFLP screening. In a first step, two sets of nearly isogenic lines for nematode resistance versus susceptibility were analyzed by AFLP fingerprinting using the following primers:
*Pst*I-primers: 5'-GACTGCGTACATGCAGNN-3'
*MseI*-primers: 5'-GATGAGTCCTGAGTAANNN-3'
The N's indicate the variable selective nucleotides. In the AFLP screening all 16 primers possible for the *PstI*-primer and all 64 primers possible for the *Mse*I-primer were used on the two sets of nearly isogenic lines, giving a total of 16 x 64 = 1024 tested primer combinations. Upon analysis of all the AFLP fingerprints a total of 30 candidate AFLP markers linked to the *Mi* resistance gene were identified. These candidate markers were subsequently tested on a panel of nematode resistant and nematode susceptible tomato lines for confirmation and distance of linkage to the *Mi* locus. The *Mi* resistance gene was introgressed in the cultivated tomato in 1944 from *Lycopersicon peruvianum*. Modern nematode resistant tomato lines have been subjected to numerous cycles of crossing expected to result in a small introgressed region from *Lycopersicon peruvianum* with the *Mi* resistance gene. Testing of the candidate AFLP markers on these modern tomato genotypes is expected to be a good test for assessing close linkage to the *Mi* locus. A panel of 7 resistant and 11 susceptible tomato genotypes was tested with the candidate AFLP markers. A total of 20 AFLP markers appeared to be present in all resistant lines and absent in all susceptible lines and are referred to as *Mi* linked AFLP markers.

Next, four of the AFLP markers were screened on a high molecular weight genomic library. The cloning of very large segments of DNA as large artificial chromosomes in yeast has become an essential step in isolating genes via positional cloning. The cloning capacity of the YAC vector allows the isolation of DNA fragments up to one million base pairs in length. The tomato line *Lycopersicon esculentum* E22, homozygous for the *Mi* locus, was used as source DNA to construct a YAC library. We obtained a YAC library containing 3840 clones with an average insert size of 520 Kb, representing approximately 2.2 genome equivalents of the tomato genome. Three positive YAC clones were obtained after the AFLP screening with the *Mi* linked AFLP markers: 1/1084, 1/1172 and 2/1256. Subsequently, the presence of all *Mi* linked AFLP markers was determined in the 3 YAC clones. All markers appeared present in one or more of the 3 YAC clones, which allowed a first positioning of the various *Mi* linked AFLP markers. The AFLP data indicated that the 3 YAC clones constituted an overlapping contig of approximately 1.4 Mb (see Figure 1).

To determine the physical size of the *Mi* locus comprising the *Mi* linked AFLP markers and comprised in YAC clones 1/1084, 1/1172 and/or 2/1256 a long-range restriction map of the YAC contig was constructed. This defined a DNA segment comprising the *Mi* locus of about 700 kb on which all the *Mi* linked AFLP markers were located (see Figure 1).

A size of 700 kb is still too large for direct localization of the *Mi* resistance gene. Such large inserts cannot be transformed into plant cells directly. Therefore, a cosmid library was constructed of the yeast strain containing YAC 1/1172 and a cosmid library was constructed of the yeast strain containing YAC 2/1256 using cosmid vectors which are suitable for *Agrobacterium* mediated transformation. The size of this binary cosmid vector amounts 29 kb and is shown schematically in Figure 2. The cloning capacity of this binary cosmid vector, using phage lambda packaging extract is within the range of 9 to 24 kb. Two banks of approximately 250,000 cosmid clones each were obtained from size fractionated yeast DNA. The cosmid banks were screened by colony hybridization using as probes labeled restriction fragments of the YACs. Positive cosmids clones were identified and in addition, the cosmids were grouped into seven defined regions covering the *Mi* region.

In the following step the set of cosmids of the seven defined regions were fingerprinted using restriction fragment amplification to determine their relative order. A cosmid contig covering a DNA segment of approximately 700 kb could be constructed. Subsequently, the presence of the *Mi* linked AFLP markers in this cosmid contig was determined. A physical map of the DNA segment comprising the *Mi* resistance gene with the positions of the various *Mi* linked AFLP markers was obtained (see Figure 3).

A total of 96 overlapping cosmids together constituted the DNA segment comprising the *Mi* resistance gene. Complementation analysis to identify the *Mi* resistance gene with such a large set of cosmids is a very laborious task. Therefore, the position of the *Mi* resistance gene on the cosmid contig was determined using mutant tomato lines. These mutant lines are members from a family originating from a common ancestor and contained a wild-type (nematode resistant) *Mi* genotype but a mutant nematode susceptible phenotype. Upon analysis with the set of *Mi* linked AFLP markers on a large number of these mutant lines three *Mi* linked AFLP markers appeared to be absent in most mutants. These AFLP markers, therefore showed a good correlation between the AFLP *Mi* genotype and the *Mi* phenotype, in contrast to all other 17 AFLP markers. Two of these AFLP markers, PM14 and PM25 were adjacent, and the region around these markers was assumed to be the most likely position for the *Mi* resistance gene. A set of 6 overlapping cosmids defining a DNA segment of approximately 50 kb around AFLP markers PM14 and PM25 was selected for complementation analysis (see Figure 4).

The final step in the identification of the *Mi* resistance gene via positional cloning is the complementation of the corresponding susceptible phenotype. The 6 cosmids from the candidate *Mi* region were introduced in Agrobacterium tumefaciens through conjugative transfer in a tri-parental mating. The presence of the cosmid in the *A. tumefaciens* strains was determined comparing various restriction enzyme patterns as well as DNA fingerprints from the *A. tumetaciens* strains with the *E.coli* strain containing the cosmid. Only those *A. tumefaciens* cultures harbouring a cosmid with the same DNA pattern as the corresponding *E. coli* culture were used to transform a susceptible tomato line. A susceptible tomato line was transformed with cosmids Mi-32, Mi-30, Mi-11, Mi-18, Mi-01 and Mi-14 using standard transformation methods.

Roots of *in vitro* grown transformed R₀ plants were tested for disease symptoms in order to identify cosmids with the resistance gene. Root explants were transferred onto solidified medium in petri dishes and inoculated with ten galls from an axenic nematode culture of the root-knot nematode *Meloidogyne incognita*. Disease symptoms are scored six weeks after inoculation. A transgenic plant is considered resistant when no galls are visible on its root culture. A transgenic plant is considered susceptible when at least two galls have been induced on its root culture. The observations of the disease assay revealed that 2 cosmids were able to complement the susceptible phenotype. The presence of the AFLP marker PM14 in the resistant R₀ plants indicated that the genomic insert present in cosmids Mi-11 and Mi-18 is also present in the R₀ plants and is involved in conferring the R₀ plants resistant to *Meloidogyne incognita*.

Finally, the inserts in cosmids Mi-11 and Mi-18 were further characterized. Sequencing analysis revealed a large open reading frame of 3621 nucleotides. The DNA sequence is listed in Figure 5.

For the identification and isolation of homologous sequences falling within the scope of the present invention, genomic and cDNA libraries were screened with the coding sequence of the *Mi* resistance gene as a probe under stringent hybridization conditions. Positive clones were isolated and used for complementation analysis.

Cosmid Mi-11 has been deposited on August 5, 1996 as plasmid pKGMi-11 at Centraalbureau voor Schimmelcultures at Baarn, The Netherlands, under deposit number CBS 822.96.

Cosmid Mi-18 has been deposited on August 5, 1996 as plasmid pKGMi-18 at Centraalbureau voor Schimmelcultures at Baarn, The Netherlands, under deposit number CBS 821.96.

The following examples will provide a further illustration of the present invention which is nevertheless not limited to these examples.

### EXAMPLES

### EXAMPLE 1: DISEASE ASSAY

An axenic culture of the root-knot nematode *Meloidogyne incognita* is maintained on sterile roots of the tomato cultivar Moneymaker. The root cultures are grown on solidified B5 medium (Gamborg *et al* 1968, Experimental Cell Research **50**: 151-158) with 2% saccharose and without hormones.
Root explants (1-5 cm), derived from *in vitro* grown transgenic tomato plants are transferred onto the solidified B5 medium mentioned above to start root cultures. At the same time each root explant is inoculated with ten galls from the axenic nematode culture. The galls are placed a few centimetres from the root explant. The Petri dishes with the roots and galls are incubated in the dark at 25°C. After four to six weeks the level of infection is determined by counting the number of galls formed on the root cultures.
The evaluation for resistance/susceptibility to *M. incognita* is as follows: A transgenic plant is considered resistant when no galls are visible on its root culture. A transgenic plant is considered susceptible when at least two gals have been induced on its root culture.

### EXAMPLE 2: IDENTIFICATION OF AFLP MARKERS LINKED TO A DNA SEGMENT COMPRISING THE Mi RESISTANCE GENE

### Tomato lines (Lycopersicon esculentum)

A total of 9 tomato lines resistant to *Meloidogyne incognita* and 13 tomato lines susceptible to *M. incognita* were used to identify AFLP markers. Initially the AFLP screening was performed on two sets of nearly isogenic lines 83M-R (resistant) and 83M-S (susceptible), and Motelle (resistant) and Mobox (susceptible). The candidate markers resulting from this first screening were confirmed by a second screening on 7 *M. incognita* resistant and 11 *M. incognita* susceptible lines.
Two sets of nearly isogenic lines :

| | | | |
|---|---|---|---|
| 1. | 83M-R | resistant | De Ruiter Zonen C.V., Bergschenhoek, The Netherlands (hereinafter "De Ruiter") |
| 2. | 83M-S | susceptible | De Ruiter |
| 3. | Motelle | resistant | INRA, Montfavet, France |
| 4. | Mobox | susceptible | INRA, Montfavet, France |

The 7 *M. incognita* resistant lines and 11 *M. incognita* susceptible lines for confirmation :

| | | | |
|---|---|---|---|
| 5. | DR30 | resistant | De Ruiter |
| 6. | DR17 | resistant | De Ruiter |
| 7. | E22 | resistant | Enza Zaden, de Enkhuizer Zaadhandel B.V., Enkhuizen, The Netherlands (hereinafter "Enza Zaden") |
| 8. | E1 | resistant | Enza Zaden |
| 9. | DR6 | resistant | De Ruiter |
| 10. | DR10 | resistant | De Ruiter |
| 11. | 1872 | resistant | Royal Sluis B.V., Enkhuizen, The Netherlands (hereinafter "Royal Sluis") |
| 12. | Moneymaker | susceptible | Agricultural University Wageningen |
| 13. | DR12 | susceptible | De Ruiter |
| 14. | DR23 | susceptible | De Ruiter |
| 15. | GT | susceptible | De Ruiter |
| 16. | RZ3 | susceptible | Rijk Zwaan Zaadteelt en Zaadhandel B.V., De Lier, The Netherlands (hereinafter "Rijk Zwaan") |
| 17. | RZ5 | susceptible | Rijk Zwaan |
| 18. | E3 | susceptible | Enza Zaden |
| 19. | E7 | susceptible | Enza Zaden |
| 20. | E16 | susceptible | Enza Zaden |
| 21. | RS1 | susceptible | Royal Sluis |
| 22. | RS2 | susceptible | Royal Sluis |

### Isolation and modification of the DNA

Total tomato DNA from the 22 lines described above was isolated from young leaves as described by Bernatzki and Tanksley (Theor. Appl. Genet. **72**, 314-321). The typical yield was 50 - 100 µg DNA per gram of fresh leaf material. Template DNA for AFLP analysis with the enzyme combination *Pst*I-*Mse*I was prepared as described by Zabeau and Vos (European Patent Application, EP 0534858), and is described briefly below:
0.5 µg of tomato DNA was incubated for 1 hour at 37°C with 5 units *Pst*I and 5 units *Mse*I in 40µl 10 mM Tris.HAc pH 7.5, 10 mM MgAc, 50 mM KAc, 5 mM DTT, 50 ng/µl BSA. Next 10 µl of a solution containing 5 pMol *Pst*I-adapters, 50 pMol *Mse*I-adapters, 1 unit T4 DNA-ligase, 1 mM ATP in 10 mM Tris.HAc pH 7.5, 10 mM MgAc, 50 mM KAc, 5 mM DTT, 50 ng/µl BSA was added, and the incubation was continued for 3 hours at 37°C. The adapters are depicted below:

The structure of the *Pst*I-adapter was:

5'-CTCGTAGACTGCGTACATGCA-3'
3'-CATCTGACGCATGT-5'

The structure of the *Mse*I-adapter was: 
5'-GACGATGAGTCCTGAG-3'
3'-TACTCAGGACTCAT-5'
 Adapters were prepared by adding equimolar amounts of both strands; adapters were not phosphorylated. After ligation, the reaction mixture was diluted to 500 µl with 10 mM Tris.HCl, 0.1 mM EDTA pH 8.0, and stored at -20°C. The diluted reaction mixture is further referred to as template DNA.

### AFLP reactions

The primers used for the AFLP screening are depicted below:
*Pst*I-primers: 5'-GACTGCGTACATGCAGNN-3'
*MseI*-primers: 5'-GATGAGTCCTGAGTAANNN-3'
The N's in the primers indicate that this part of the primers was variable. In the AFLP screening all 16 possible primers were used for the *Pst*I-primer and all 64 possible primers were used for the *Mse*I-primer. This gave a total of 16 x 64 combinations of *Pst*I- and *Mse*I-primers, is 1024 primer combinations. All 1024 primer combinations were used in the AFLP screening for *Mi* linked AFLP markers. The AFLP reactions were performed in the following way:
AFLP reactions employed a radio-actively labelled *Pst*I-primer and a non-labelled *Mse*I-primer. The *Pst*I-primers were end-labelled using (γ-³³P)ATP and T4 polynucleotide kinase. The labelling reactions were performed in 50 µl 25 mM Tris.HCl pH 7.5,10 mM MgCl₂, 5 mM DTT, 0.5 mM spermidine.3HCl using 500 ng oligonucleotide primer, 100 µCi (γ-³³P)ATP and 10 units T4 polynucleotide kinase. For AFLP analysis 20 µl reaction mixture were prepared containing 5 ng labeled *Pst*I-primer (0.5 µl from the labeling reaction mixture), 30 ng *Mse*I-primer, 5 µl template-DNA, 0.4 units Taq-polymerase, 10 mM Tris.HCl pH 8.3, 1.5 mM MgCl₂, 50 mM KCl, 0.2 mM of all 4 dNTPs. AFLP reactions were performed using the following cycle profile: a 30 seconds DNA denaturation step at 94°C, a 30 seconds annealing step (see below), and a 1 minute extension step at 72°C. The annealing temperature in the first cycle was 65°C, was subsequently reduced each cycle by 0.7°C for the next 12 cycles, and was continued at 56°C for the remaining 23 cycles. All amplification reactions were performed in a PE-9600 thermocycler (Perkin Elmer Corp., Norwalk, CT, USA).

### Gel analysis of AFLP reaction products

After amplification, reaction products were mixed with an equal volume (20 µl) of formamide dye (98% formamide, 10 mM EDTA pH 8.0, and bromo phenol blue and xylene cyanol as tracking dyes). The resulting mixtures were heated for 3 minutes at 90°C, and then quickly cooled on ice. 2 µl of each sample was loaded on a 5% denaturing (sequencing) polyacrylamide gel (Maxam and Gilbert, Methods in Enzymology **65**, 499-560). The gel matrix was prepared using 5% acrylamide, 0.25% methylene bisacryl, 7.5 M urea in 50 mM Tris/50 mM Boric acid/1 mM EDTA. To 100 ml of gel solution 500 µl of 10% APS and 100 µl TEMED was added and gels were cast using a SequiGen 38 x 50 cm gel apparatus (Biorad Laboratories Inc., Hercules, CA, USA). Sharktooth combs were used to give 97 lanes on the SequiGen gel units. 100 mM Tris/100 mM Boric acid/2 mM EDTA was used as running buffer. Electrophoresis was performed at constant power, 110 Watts, for approximately 2 hours. After electrophoresis, gels were fixed for 30 minutes in 10% acetic acid dried on the glass plates and exposed to Fuji phospho image screens for 16 hours. Fingerprint patterns were visualized using a Fuji BAS-2000 phospho image analysis system (Fuji Photo Film Company Ltd, Japan).

### AFLP screening for linked markers

An AFLP screening was performed using all possible 1024 *Pst*I-*Mse*I primer combinations on the two sets of nearly isogenic lines. The aim was to identify AFLP markers present in both resistant lines and absent in both susceptible lines. AFLP gels contained the AFLP fingerprints of 24 primer combinations of the 4 isogenic lines, giving a total of 43 gels. A total of 30 AFLP markers were identified present in both resistant lines and absent in both susceptible lines. These markers are referred to as candidate *Mi* linked AFLP markers.
Next, AFLP reactions were performed to determine the presence of the 30 candidate markers on the 7 resistant and 11 susceptible tomato lines. Of the 30 candidate markers 20 markers appeared to be present in the 7 resistant lines and absent in the 11 susceptible lines. These 20 markers were used in further studies to map the *Mi* resistance gene. The primer combinations required to identify the 20 *Pst*I-*Mse*I markers are depicted in Table 1. In the column with the primer combinations, "*Pst*I-" refers to the sequence 5'-GACTGCGTACATGCAG-3' and "*Mse*I-" refers to the sequence 5'-GATGAGTCCTGAGTAA-3'. For example, marker PM14 can be identified using the *Pst*I-primer having the following sequence: 5'-GACTGCGTACATGCAGGA-3', and the *Mse*I-primer having the following sequence: 5'-GATGAGTCCTGAGTAATCT-3.'

**TABLE 1**

| marker | primer combination with selective extensions (NN/NNN) |
|---|---|
| PM02 | *Pst*I-AT /*Mse*I-AAA |
| PM07 | *Pst*I-AA /*Mse*I-TAC |
| PM08 | *Pst*I-CT /*Mse*I-ACT |
| PM10 | *Pst*I-CA /*Mse*I-TCT |
| PM11 | *Pst*I-TA /*Mse*I-TGA |
| PM13 | *Pst*I-GA /*Mse*I-ATC |
| PM14 | *Pst*I-GA /*Mse*I-TCT |
| PM15 | *Pst*I-GT /*Mse*I-GAC |
| PM16 | *Pst*I-GT /*Mse*I-TCT |
| PM17 | *Pst*I-AT /*Mse*I-AAG |
| PM18 | *Pst*I-AT /*Mse*I-TAG |
| PM19 | *Pst*I-GG /*Mse*I-ATT |
| PM20 | *Pst*I-TG /*Mse*I-AAT |
| PM21 | *Pst*I-TG /*Mse*I-TTT |
| PM22 | *Pst*I-TG /*Mse*I-GCT |
| PM23 | *Pst*I-GT /*Mse*I-GAA |
| PM24 | *Pst*I-AA /*Mse*I-CTG |
| PM25 | *Pst*I-AC /*Mse*I-GTG |
| PM27 | *Pst*I-AA /*Mse*I-CTA |
| PM29 | *Pst*I-TA /*Mse*I-GGA |

### EXAMPLE 3: CONSTRUCTION AND SCREENING OF A TOMATO YAC LIBRARY

### Material

The tomato line *Lycopersicon esculentum* E22 (Enza Zaden) homozygous for the *Mi* locus, was used as source DNA to construct a YAC library. Protoplasts were isolated from the leaves of *in vitro* shoots which were two to three weeks old as described by Van Daelen *et al* (Plant Mol. Biol. **12**, 341-352).
Viable protoplasts (concentration of 50 million protoplasts per ml) were collected and mixed with an equal volume of agarose (1%, Seaplaque, FMC Bioproducts, Rockland, Maine, USA) to form a plug. The protoplasts embedded into the plugs were lysed with lysis mix (0.5 M EDTA, 1% N-Laurylsarcosinate and 1 mg/ml proteinase K, pH = 8.0). After lysis, the plugs were stored at 4°C in storage buffer (fresh lysis mix) until used. Approximately 3 million protoplasts per plug, to obtain about 4.5 µg of chromosomal DNA were used for further studies. Plasmid pYAC4 containing an unique *Eco*RI cloning site was used as cloning vector and the yeast strain AB1380 was used as a host (Burke *et al*, Science **236**, 806-812).

### YAC library construction

High molecular weight DNA isolation, partial digestion with *Eco*RI in the presence of *Eco*RI methylase, ligation of vector arms to genomic DNA, size selection by pulsed field gel electrophoresis and transformation of the yeast host was performed as described by Burke *et al*, (Science **236**, 806-812) and Larin *et al*, (Proc Natl Acad Sci USA **88**, 4123-4127).
All standard manipulations were carried out as described in Molecular cloning: a laboratory manual by Sambrook *et al*, (Cold Spring Harbor Laboratory Press). 3840 clones with a average insert size of 520 kb, which corresponds to 2.2 genome equivalents were finally obtained and the individual clones were stored in 40 96-wells microtiter plates containing 75 µl YPD solution (1% yeast extract, 2% peptone and 2% dextrose).

### Screening YAC library

To reduce the number of samples handled, the cells of one 96-well microtiter plate were pooled (a platepool) and used for DNA isolation as described by Ross *et al* (Nucleic Acids Res., **19**, 6053). The 2.2 genome equivalent tomato YAC library consists of 40 96-wells microtiter wells and as a result DNA of the 40 platepools were screened with the AFLP markers PM10, PM13, PM21 and PM25 using the AFLP protocol as described in Example 2. PM10, PM13, PM21 and PM25 were selected to screen the YAC platepools because these markers do not interfere with the background bands of the yeast strain AB1380. Three positive platepools out of the 40 were identified with these four AFLP markers as shown in Table 2. Subsequently, a secondary screening with the four AFLP markers (PM10, PM13, PM21 and PM25) of the 96 individual YAC clones of each plate was employed to find the correct address of the YAC clones. Three individual YAC clones were identified, designated 1/1084, 1/1172 and 2/1256 (Table 2). Subsequently, the three individual YAC clones were analyzed with the remaining AFLP markers. All of the identified markers PM02 to PM29 were present on one or more these three YAC clones (Table 3). The size of the YAC clone was determined by Pulse-field gel electrophoretic (PFGE) analysis using contour-clamped homogeneous electric field (CHEF; Chu *et al* Science, **235**, 1582-1585) and appeared to be 470 kb (1/1084), 570 kb (1/1172), and 500 kb (2/1256) respectively.

**TABLE 2**

| Platepool nr | PM10 | PM13 | PM21 | PM25 | YAC detected (size in kb) |
|---|---|---|---|---|---|
| 2 | - | - | + | - | YAC 1/1172 (570 kb) |
| 16 | + | + | - | + | YAC 2/1256 (500 kb) |
| 4 | - | + | - | - | YAC 1/1084 (470 kb) |

**TABLE 3**

| Marker | 1/1172 | 2/1256 | 1/1084 |
|---|---|---|---|
| PM02 | - | - | + |
| PM07 | - | + | - |
| PM08 | - | + | + |
| PM10 | - | + | - |
| PM11 | - | + | - |
| PM13 | - | + | + |
| PM14 | + | + | - |
| PM15 | - | + | - |
| PM16 | + | - | - |
| PM17 | - | + | - |
| PM18 | - | + | + |
| PM19 | - | + | - |
| PM20 | - | + | - |
| PM21 | + | - | - |
| PM22 | - | + | + |
| PM23 | - | + | - |
| PM24 | - | + | - |
| PM25 | - | + | - |
| PM27 | - | + | - |
| PM29 | - | + | - |

### EXAMPLE 4: CONSTRUCTION OF A LONG RANGE PHYSICAL MAP OF THE Mi YAC CONTIG AND LOCATION OF THE AFLP MARKERS

The 3 YAC clones 1/1172, 2/1256 and 1/1084 were subjected to partial digestion with increasing concentration of the restriction enzymes *Sfi*I and *Bss*HII. The samples were fractionated by PFGE, transferred to a Gene Screen Plus membrane (DuPont NEN, Boston, MA, USA) and assayed by hybridization using end-adjacent sequence probes according to the protocol for indirect end-label mapping as described by Burke *et al* (Science **236**, 806-812). A physical map of YAC 1/1172, 2/1256 and 1/1084 for the enzymes *Sfi*I and *Bss*HII could be constructed as shown in Figure 1. The overlap between the various YAC clones was determined by Southern blot analysis using the obtained restriction fragments as a probe on digest of the three YAC clones. A YAC contig with a size of 1.4 Mb could be constructed. In order to isolate the YAC fragments the digests were run on PFGE. Digestion of YAC 1/1172 with *Sfi*I resulted in two fragments (200 Kb and 370 Kb). Digestion of YAC 2/1256 with *Bss*HII resulted in four fragments (40 Kb, 90 Kb, 110 Kb and 260 Kb) whereas digestion of YAC 1/1084 with *Bss*HII gave two fragments with a size of 70 and 400 kb. As a result the 1.4 Mb YAC contig could be dissected into 8 regions corresponding to the 8 restriction fragments obtained from the three YAC clones, covering the complete *Mi* region and adjacent sequences.
To position the various AFLP markers within these 8 regions on the physical map, the AFLP markers were used as hybridization probes on the partial and complete *Sfi*I and *Bss*HII digests of YAC clones 1/1172, 2/1256 and 1/1084. Therefore, each AFLP marker fragment was excised from the dried gel and eluted by means of diffusion in a buffer containing 0.5 M ammonium acetate, 10 mM magnesium acetate, 1 mM EDTA (pH=8.0), 0.1% SDS, re-amplified with the corresponding unlabelled AFLP primers and, subsequently labelled with ³²P according to the random primer method of Feinberg and Vogelstein (Anal. Biochem. **132**, 6-10). Each AFLP marker could be assigned to one or more of the eight regions as outlined in Table 4 and Figure 1.

**TABLE 4**

| YAC fragment | *Mi* linked AFLP markers detected by hybridization |
|---|---|
| 200 kb *Sfi*I-fragment 1/1172 | - |
| 370 kb *Sfi*I-fragment 1/1172 | PM14, PM16, PM21 |
| 260 kb *Bss*HII-fragment 2/1256 | PM10, PM11, PM17, PM19, PM23, PM24, PM29 |
| 90 kb *Bss*HII-fragment 2/1256 | PM07, PM27 |
| 110 kb *Bss*HII-fragment 2/1256 | PM08, PM13, PM14, PM15, PM20, PM22, PM25 |
| 40 kb *Bss*HII-fragment 2/1256 | PM18 |
| 70 kb *Bss*HII-fragment 1/1084 | PM08, PM13, PM22 |
| 400 kb *Bss*HII-fragment 1/1084 | PM02, PM18 |

### EXAMPLE 5: CONSTRUCTION OF A COSMID LIBRARY OF YAC CLONES 1/1172 AND 2/1256

### Material

The binary cosmid vector pJJ04541 is a derivative of pJJ1881 (Jones *et al*, Transgenic Research **1**, 285-297) and is based on plasmid pRK290 containing the tetracyclin resistance gene for selection in *Escherichia coli* and *Agrobacterium tumefaciens*. Into the unique *Eco*RI site of pRK290, T-DNA carrying sequences (LB; left border repeat, RB signifies the right border repeat) that flank
- the cos site of bacteriophage lambda
- the neomycin phosphotransferase gene (Beck *et al*, Gene **19**, 327-336) whose expression is driven by the cauliflower mosaic virus 35S promoter sequence (Odell *et al*, Mol Gen Genet **223**, 369-378), and
- the pBluescript (Stratagene, La Jolla, California, USA) polylinker sequence.
The size of pJJ04541 amounts 29 kb and is shown schematically in Figure 2. The cloning capacity of this binary cosmid vector, using phage lambda packaging extracts is within the range of 9 to 24 kb.

### Library construction

Total DNA of the *Saccharomyces cerevisae strain* AB1380 containing YAC 1/1172 and total DNA of the *Saccharomyces cerevisae strain* AB1380 containing YAC 2/1256 was isolated using zymolyase to make protoplasts according to Green and Olsen (Proc Natl Acad Sci USA **87**, 1213-1217).
An aliquot of both DNAs was analyzed on PFGE. Both DNA isolates appeared to have a size of ≥ 100 kb.
Approximately 15 µg of each DNA was partially digested with *Sau*3A generating molecules with an average size of 15-25 kb. Subsequently, the samples were centrifugated through a 10-35% sucrose gradient for 22 hours, 22.000 rpm at 20°C in a Beckman SW41 rotor. 0.5 ml fractions were collected using a needle pierced through the bottom of the centrifuge tube. An aliquot of these fractions was analyzed on a 0.7% agarose gel. The fractions containing DNA molecules with a size of ≈20 kb were pooled and concentrated by ethanol precipitation. Subsequently, the cohesive ends were partially filled-in with dATP and dGTP using the strategy of partial filling of 5'-extensions of DNA produced by type II restriction endonuclease as described by Korch (Nucleic Acids Res. **15**, 3199-3220) and Loftus *et al* (Biotechniques **12**, 172-176).
The binary cosmid vector pJJ04541 was digested completely with *Xho*I and the linear fragment was partially filled-in with dTTP and dCTP as described by Korch (Nucleic Acids Res. **15**, 3199-3220).
The 20-kb fragments were ligated to the cosmid vector and transduced to *E. coli* strain XL1-Blue MR (Stratagene, La Jolla, California, USA) using phage lambda Gigapack II XL packaging extracts (Stratagene, La Jolla, California, USA) as recommended by the manufacturers. Selection was performed on LB (1% bactotryptone, 0.5% bacto-yeast extract and 1% NaCl, pH 7.5) agar plates containing 10 mg/l of tetracyclin. Two banks of approximately 250.000 cosmid clones per bank were made from 2-3 µg of size fractionated yeast DNA of YAC clones 1/1172 and 2/1256 respectively.
Subsequently, these transformants were stored into the wells of microtiter plates (96-wells, 100 µl of LB medium containing 10 mg/l of tetracyclin). Replicas of the 96-well grid of cosmid clones in microtiter plates were stamped onto Gene Screen Plus membrane filters (NEN Dupont) and allowed to grow into colonies on media. Colony hybridization, as described by Sambrook *et al* (in: Molecular cloning: a laboratory manual, 1989, Cold Spring Harbor Laboratory Press), using ³²P-labelled YAC clones 1/1172 and 2/1256 revealed positive cosmids. Of about 10.000 colonies of YAC 1/1172 approximately 200 positive cosmid clones were identified. Of about 20.000 colonies of YAC 2/1256 300 positive cosmid clones were identified.

### EXAMPLE 6: FINE MAPPING OF THE Mi RESISTANCE GENE SEGMENT AND POSITIONING OF THE AFLP MARKERS

### Dividing the cosmids in defined regions

In order to divide the cosmids into seven defined regions, the 200 positive cosmid clones of YAC 1/1172 and the 300 positive cosmid clones of YAC 2/1256 were hybridized with 7 of the 8 restriction fragments (YAC fragments) as outlined in Example 4 (see Table 4 and Figure 1). Positive cosmids for each of the 7 YAC fragments were identified. In addition, cosmids could be identified which reacted positively with the overlapping restriction fragments of the two different YAC clones.

### Construction of a cosmid contig of the Mi resistance gene segment

In order to construct a cosmid contig of all the positive identified cosmids in the various defined regions restriction fragment amplification was used. Approximately 500 ng of each cosmid was used for template preparation and the primers in the amplification of restriction fragments were a *Eco*RI-primer 5'-GACTGCGTACCAATTC-3' having no selective nucleotides and a *Mse*I-primer 5'-GATGAGTCCTGAGTAA-3' having no selective nucleotides according to the method as described in Example 2. The *Eco*RI-primer was labelled at the 5' end and all the 500 cosmids were amplified using *Eco*RI/*Mse*I-primer set. The DNA fingerprints contained about 8 to 20 amplified fragments. Sets of cosmids containing amplified fragments of identical size were selected from each region and were rerun on polyacrylamide gels as described in Example 2 until a contiguous array of all the amplified fragments throughout the defined regions could be constructed. In addition, the cosmid contig of one region was aligned with the adjacent regions in order to construct a cosmid contig of the *Mi* locus. In this way a cosmid contig of 96 cosmids was constructed spanning the *Mi* locus of approximately 800 kb.

### Detailed positioning of the Mi linked AFLP markers on the cosmid contig

In order to position the 20 *Mi* linked AFLP markers on the cosmid contig, the 96 cosmids were digested with *Pst*I followed by Southern blot analysis according to Southern, J. Mol. Biol. **98**, 503-515.
The AFLP markers were used as hybridization probes as described in Example 4 on the Southern blot of the 96 *Pst*I digests of the cosmids. The exact position of the *Mi* linked AFLP markers, except marker PM02, is outlined in Figure 3A.

### EXAMPLE 7: GENETIC ANALYSIS OF Mi MUTANTS

A family of mutant tomato lines was made available through Enza Zaden. These lines were derived from a F₁ hybrid heterozygous for the *Mi* resistance gene and heterozygous for the *Aps-1* gene (encoding acid phosphatase-1), which is very closely linked to *Mi* (Stevens and Rick, 1986, in: The Tomato Crop, Atherton & Rudich edit., Chapman and Hall, p. 35-109). Different alleles of the *Aps-1* gene can be determined by isozyme analysis (Vallejos, 1983, in: Isozymes in plant genetics and breeding, Tanksley and Orton edit., part A, Elsevier, Amsterdam, 469-515) The *Aps-1*¹ allele originates from *L. perinvianum* and has been introgressed into several nematode resistant tomato genotypes by co-segregation with the *Mi* resistance gene. A scheme of these mutant lines is depicted below: 
F1 - hybrid (heterozygous Aps-1, Mi resistant phenotype)
↓ selfed
F2 - lines (segregating Aps-1 1:2:1, segregating Mi resistance 3:1)
↓ selfing of heterozygous (Aps-1) F2 plants
F3 - lines (segregating Aps-1 1:2:1, segregating Mi resistance 3:1)
↓ selfing of heterozygous (Aps-1) F3 plants
F4 - lines (segregating Aps-1 1:2:1, segregating Mi resistance 3:1)
↓ selfing of heterozygous (Aps-1) F4 plants
F5 - lines (segregating Aps-1 1:2:1, Mi susceptible)
↓ selfing of heterozygous (Aps-1) F5 plants
F6 - lines (segregating Aps-1 1:2:1, Mi susceptible)
↓ selfing of homozygous (Aps-1^1) F6 plants
F7 - lines (all Aps-1^1, Mi susceptible)
↓ selfing of homozygous (Aps-1^1) F7 plants
F8 - lines (all Aps-1^1, Mi susceptible)
 In the F₁, F₂, F₃ and F₄ lines of this family the presence of the *Aps-1*¹ allele correlates with the *Mi* resistant phenotype, whereas absence of the *Aps-1*¹ allele correlates with the *Mi* susceptible phenotype. In the F₅ and subsequent progenies this correlation is lost: all plants are susceptible to nematodes regardless of the *Aps-1* alleles.
Twenty individuals from each F₂, F₃, F₄, F₅, F₆, F₇ and F₈ generation were tested for nematode resistance, for presence of the *Aps-1* allele and presence of the *Mi* linked AFLP markers. Nematode testing of seedlings was performed in soil contaminated with root galls of *M. incognita*. The nematode resistance results were as indicated in the above scheme: 3:1 segregation in F₂, F₃ and F₄ plants and susceptibility in F₅ and progeny populations. Most of the *Mi* linked AFLP markers indicated an identical *Mi* genotype as the *Aps-1* isozyme marker. However, 3 of the AFLP markers PM14, PM16 and PM25 appeared to segregate with the *Mi* phenotype: In most F₅, F₆, F₇ and F₈ plants the *Mi* susceptibility was indicated by the absence of these markers. The AFLP markers PM14, PM16 and PM25 showed a correlation between the AFLP *Mi* genotype and *Mi* phenotype in the mutants. Markers PM14 and PM25 are adjacent on the physical map as shown in Figure 3B, and therefore, it was postulated that the region surrounding these AFLP markers was a good candidate to comprise the *Mi* resistance gene.

### EXAMPLE 8: PHYSICAL MAP OF THE OVERLAPPING COSMID CLONES COMPRISING THE Mi RESISTANCE GENE

The identification of cosmids hybridizing with the *Mi* linked AFLP markers PM14 and PM25 was performed in Example 6. PM14 identifies cosmids Mi-11, Mi-18 and Mi-01 whereas PM25 identifies cosmids Mi-18 and Mi-01.
Subsequently, a small cosmid array around cosmids Mi-11, Mi-18 and Mi-01 was selected from the cosmid contig described in Example 6. A contig of 6 cosmids comprising the 3 identified cosmids and the adjacent cosmids, was selected. These 6 cosmids are Mi-32, Mi-30, Mi-11, Mi-18, Mi-01 and Mi-14. In order to make a physical fine map of these 6 cosmids, the DNA samples of the cosmid contig were digested with *Pst*I followed by electrophoresis on a 0.8%-agarose gel. The physical overlap between the various cosmids could be determined. Combining these data with the data obtained about the detailed positioning of the *Mi* linked AFLP markers on the cosmid contig (see Example 6) a physical fine map with the location of PM14 and PM25 could be constructed as shown in Figure 4. The cosmid contig around the AFLP markers PM14 and PM25 was calculated to be approximately 50 kb.

### EXAMPLE 9: TRANSFORMATION

### Transfer of cosmids to Agrobacterium tumefaciens

The cosmid clones Mi-32, Mi-30, Mi-11, Mi-18, Mi-01, Mi-14 and the control cosmid pJJ04541 were introduced in *Agrobacterium tumefaciens* through conjugative transfer in a tri-parental mating with helper strain HB101 (pRK2013) essentially according to Deblaere *et al* (Methods in Enzymology **153**, 277-292). *E.coli* were grown in LB medium (1% bacto-tryptone, 0.5% bacto-yeast extract and 1% NaCl, pH 7.5) supplemented with 5 mg/l tetracyclin at 37°C. The helper strain HB101 (pRK2013) was grown under identical conditions in LB medium supplemented with 100 mg/l kanamycin sulphate.
*Agrobacterium tumefaciens* strain AGL1 (Lazo *et al*, Bio/Technology, **9**, 963-971, 1991) was grown in LB medium supplemented with 100 mg/l carbenicillin at 28°C.
Overnight cultures were diluted 1:100 in LB medium without any antibiotics and after 6 hours of growth, 0.1 ml each of the Agrobacterium culture, the helper strain culture and a cosmid strain culture were mixed and plated on LB agar plates without antibiotics. After overnight incubation at 28°C, the mixture was plated on LB medium agar plates containing 100 mg/l carbenicillin and 10 mg/l tetracyclin to screen for transconjugants. Plates were incubated for 3-4 days at 28°C. Two serial passages through selective agar plates were performed to select for single transconjugant *Agrobacterium* colonies.

### Characterization of A. tumefaciens transconjugants

Small-scale cultures were grown from selected colonies and grown in LB medium containing 10 mg/l tetracyclin. Plasmid DNA was isolated by alkaline lysis using the method as described by Ish-Horowicz et al (Nucl. Acids Res. **9**, 2989-2997, 1981), and digested with *Bgl*II using standard techniques. In addition, restriction fragment amplification on miniprep DNA of *A. tumefaciens* was performed using the enzyme combination *Eco*RI/*Mse*I and primers having no selective nucleotide as described in Example 6. Subsequently, the *Bgl*II restriction enzyme pattern as well as the DNA fingerprint of the *A. tumefaciens* transconjugant were compared with those of miniprep DNA of the *E. coli* strain containing the cosmid. Only those *A. tumefaciens* transconjugants harbouring a cosmid with the same DNA pattern as the corresponding *E. coli* culture were used to transform a susceptible tomato line.

### Transformation of a susceptible tomato line

Seeds of the susceptible tomato line 52201 (Rijk Zwaan) were surface-sterilized in 2% sodium hypochlorite for 10 minutes, rinsed three times in sterile distilled water, and placed on germination medium (consisting of half-strength MS medium according to Murashige and Skoog, Physiol. Plant. **15**, 473-497, with 1% (w/v) sucrose and 0.8% agar) in glass jars or polypropylene culture vessels. They were left to germinate for 8 days. Culture conditions were 25°C, a photon flux density of 30 µmol.m⁻².s⁻¹ and a photoperiod of 16 /24 h.
Transformation of tomato was performed according to Koornneef *et al*, In: Tomato Biotechnology, 169-178, Alan R. Liss, Inc., and is described briefly below. Eight day old cotyledon explants were precultured for 24 hours in Petri dishes containing a feeder layer of *Petunia hybrida* suspension cells plated on MS20 medium (culture medium according to Murashige and Skoog, Physiol. Plant. **15**, 473-497 with 2% (w/v) sucrose and 0.8% agar) supplemented with 10.7 µM α-naphthaleneacetic acid and 4.4 µM 6-benzylaminopurine. The explants were then infected with the diluted overnight culture of *Agrobacterium tumefaciens* containing the cosmid clones Mi-32, Mi-30, Mi-11, Mi-18, Mi-01 and Mi-14 or the cosmid vector pJJ04541 for 5-10 minutes, blotted dry on sterile filter paper and cocultured for 48 hours on the original feeder layer plates. Culture conditions were as described above. Overnight cultures of *Agrobacterium tumefaciens* were diluted in liquid MS20 medium (medium according to Murashige and Skoog (1962) with 2% (w/v/) sucrose, pH 5.7) to an O.D.₆₀₀ of 0.8.
Following the cocultivation, the cotyledon explants were transferred to Petri dishes with selective medium consisting of MS20 supplemented with 4.56 µM zeatin, 67.3 µM vancomycin, 418.9 µM cefotaxime and 171.6 µM kanamycin sulphate, and cultured under the culture conditions described above. The explants were subcultured every 3 weeks onto fresh medium. Emerging shoots were dissected from the underlying callus and transferred to glass jars with selective medium without zeatin to form roots. The formation of roots in a medium containing kanamycin sulphate was regarded as an indication of the transgenic nature of the shoot in question. Truly transgenic regenerants were propagated *in vitro* by subculturing the apical meristem and auxiliary buds into glass jars with fresh selective medium without zeatin.

### EXAMPLE 10: COMPLEMENTATION ANALYSIS

### Identification of cosmids with the Mi resistance gene by screening for resistance in roots of transformed plants

Roots of *in vitro* grown transformed R₀ plants have been subjected to the disease assay as described in Example 1. From each transformant two root explants have been assayed. In total 72 R₀ plants of 7 different cosmid transformations have been tested; 6 cosmids carrying tomato insert DNA and one cosmid, pJJ04541, is without insert DNA. The results are shown in Table 1. Sixty three transgenic R₀ plants appeared susceptible, because galls had been formed on at least one of the two root cultures. Nine R₀ plants scored resistant, because no galls could be found on the root cultures. Seven resistant plants had been derived from transformation with cosmid Mi-11, while two resistant plants had been derived with cosmid Mi-18, that is overlapping for a great part with cosmid Mi-11. The cosmids Mi-11 and Mi-18 were used for further molecular analysis.

**TABLE 1**

| Cosmid | R₀ plants | |
|---|---|---|
| | Resistant | Susceptible |
| Mi-32 | 0 | 8 |
| Mi-30 | 0 | 11 |
| Mi-11 | 7 | 4 |
| Mi-18 | 2 | 8 |
| Mi-01 | 0 | 10 |
| Mi-14 | 0 | 15 |
| pJJ04541 | 0 | 7 |

### Molecular analysis of the transformed plants with a resisiant phenotype

To demonstrate that the resistant phenotype of transgenic R₀ plants, which were transformed with the overlapping cosmids Mi-11 and Mi-18, is determined by the genomic insert present in the various cosmids, an AFLP analysis with the AFLP marker PM14 was performed. Selective restriction fragment amplification was performed with the primer combination identifying marker PM14 for the R₀ plants transformed with cosmids Mi-11 and Mi-18. The DNA fingerprints obtained showed the presence of the marker PM14 in the resistant plants indicating that the genomic insert present in cosmids Mi-11 and Mi-18 is also present in the R₀ plants and that the two identified overlapping cosmids Mi-11 and Mi-18 comprise the *Mi* resistance gene.

The inserts in cosmids Mi-11 and Mi-18 and the inserts in the adjacent cosmids Mi-32, Mi-30 on one side and cosmids Mi-01 and Mi-14 on the other side, were further characterized. The DNA region comprising the *Mi* resistance gene based on the overlap between the cosmids Mi-11 and Mi-18, was estimated at approximately 16-18 kb. Based on the susceptibility of the R₀ plants having the insert present in cosmid Mi-30, this region could be narrowed down to approximately 12 kb. A DNA segment comprising the *Mi* resistance gene, corresponding to the region flanked by the right ends of cosmids Mi-30 and Mi-11, was sequenced (see Figure 4).

### EXAMPLE 11: NUCLEOTIDE SEQUENCE AND DEDUCED AMINO ACID SEQUENCE OF THE Mi RESISTANCE GENE FROM TOMATO

### Subcloning of the overlapping DNA segment

To determine the sequence of the overlapping DNA segment in cosmids Mi-11 and Mi-18 containing the *Mi* resistance gene, a set of random subclones with a insert size of approximately 2 kb were generated. 7.5 µg of CsCl purified DNA of cosmids Mi-11 and Mi-18 was sheared for 10 seconds at 4°C at 15% probe power (in 40 µl 10mM Tris-acetate, 10mM Mg-acetate and 50mM K-acetate) using a Misonix (Misonix Inc., Farmingdale, NY, USA) sonicator (type XL2020) with a water filled cup horn (type 431A). Subsequently, the DNA was heated for 10 minutes at 60°C and cooled to room temperature. The ends of the DNA fragments were repaired by adding 10µl of a repair mixture (10mM Tris-acetate, 10mM Mg-acetate, 50 mM K-acetate, 10U Klenow DNA polymerase, 10U T₄DNA polymerase and 2 mM of all 4 dNTP's) and followed by incubation for 30 minutes at 20°C. The sheared DNA was separated by electrophoresis on 1% Seakem GTG agarose gel (FMC Bio Products, Rockland, ME, USA). The fraction with a size of 1.8-2.2 kb was excised from the gel and subsequently the gel slice was digested with β-agarase I according to the protocol of the manufacturer (New England Biolabs Inc, Beverly, MA, USA) and the DNA was precipitated.
A modified pUC19 vector (designated pStuc) was used to clone the 1.8-2.2 kb fraction. In this vector the BamHI/SalI fragment of pUC19 was replaced by a DNA fragment containing a StuI, SpeI and SalI restriction site using two oligonucleotide primers and standard cloning techniques as described by Sambrook *et al*. (in: Molecular cloning: a laboratory manual, 1989, Cold Spring Harbor Laboratory Press). The 1.8-2.2 kb fraction was ligated at 16°C in the a StuI digested and dephosphorylated pStuc vector. The ligation mixture was subsequently transformed to Epicurian Coli XL2-Blue MRF' ultracompetent cells (Stratagene, La Jolla, CA, USA). Individual colonies were grown and stored in 384-wells microtiter plates (100 µl of LB medium containing 100 mg/l of carbenicillin).
To isolate clones representing the overlapping DNA region in cosmids Mi-11 and Mi-18 containing the *Mi* resistance gene, the 8.6 and 4.5 kb PstI fragment of cosmid clone Mi-18 (see Figure 4) as well as the AFLP marker PM14 were used as hybridization probes in colony hybridizations. Therefore, replicas of the 384-well grid of clones in microtiter plates were stamped onto Gene Screen Plus membrane filters (DuPont NEN, Boston, MA, USA) and allowed to grow into colonies on media.
Eighty four positive clones were used to isolate plasmid DNA using the alkaline lysis method as described by Ish-Horowicz *et al*. 1981, Nucl. Acids Res. **9**, 2989-2997.

### Sequence analysis

The ABI PRISM dye terminator cycle sequencing ready reaction kit was used to perform sequencing reactions in a Gene-Amp PCR system Model 9600 (Perkin-Elmer, Foster City, CA, USA). Standard M13 forward and reverse primers were used. The reaction products were analyzed on 48 cm gels of an ABI Prism 377. The DNA sequence of 84 selected clones was determined using the standard forward and reverse sequencing primers. Sequence assembly and analysis was done with the 1994 version of the STADEN sequence analysis program (Dear and Staden, 1991, Nucl. Acids Res. **19**, 3907-3911). A contiguous DNA sequence of approximately 9.9 kb nucleotides could be formed and is shown in Figure 5. A large open reading frame of 3621 nucleotides encoding a protein of 1206 amino acids could be deduced.

## Claims

1. A nucleic acid whose DNA sequence is at least part of the DNA sequence provided in figure 5 or any DNA sequence homologous thereto.

2. The nucleic acid of claim 1 which is capable, when transferred to a host plant, which is susceptible to a plant pathogen, of rendering said host plant resistant to said plant pathogen.

3. The nucleic acid of claim 1 wherein said DNA sequence homologous to the DNA sequence of figure 5 is capable, when transferred to a host plant also liable of being rendered resistant by the DNA sequence of figure 5, of also rendering it resistant to a nematode.

4. A nucleic acid according to claim 1 wherein said DNA sequence corresponds to a coding sequence starting at nucleotide 3491 and ending at nucleotide 7111 or any DNA sequence homologous thereto.

5. A nucleic acid according to claim 1 wherein said DNA sequence corresponds to a promoter sequence located 5' upstream of nucleotide 3491 or any DNA sequence homologous thereto.

6. A nucleic acid of claim 1 wherein said DNA sequence corresponds to at least part of the genomic insert present in cosmid Mi-11, or any DNA sequence homologous thereto.

7. A recombinant DNA construct comprising a nucleic acid according to any of claims 1-6.

8. A recombinant DNA construct of claim 7 in which said nucleic acid is under control of a promoter which is functional in a plant cell, said promoter being either endogenous or exogenous to said plant cell, and effective to control the transcription of said DNA sequence in such plant cells.

9. A recombinant DNA construct of claim 8 in which said promoter corresponds to a promoter sequence located 5, upstream of nucleotide 3491 as provided in figure 5, or any DNA sequence homologous thereto.

10. A vector suitable for transforming plant cells comprising a DNA construct according to any of claims 7-9.

11. Plasmid pKGMi-11 as deposited under number CBS 822.96.

12. Plasmid pKGMi-18 as deposited under number CBS 821.96.

13. Bacterial cells comprising a vector or plasmid according to any of claims 10-12.

14. Recombinant plant genome comprising, incorporated thereinto, a DNA construct according to any of claims 7-9.

15. Plant cells comprising a DNA construct according to any of claims 7-9.

16. Plant comprising plant cells according to claim 15.

17. Plant according to claim 16 which has a reduced susceptibility to nematodes.

18. Plant according to claim 17 wherein said nematode is a root-knot nematode.

19. Plant according to claim 18 wherein said root-knot nematode is *Meloidogyne incognita*.

20. Seed comprising a DNA construct according to any of claims 7-9.

21. The recombinant plant genome of claim 14, in a plant cellular environment.

22. Process for obtaining plants having reduced susceptibility to a pathogen, comprising the following steps:
i) inserting into the genome of a plant cell a DNA construct according to any of claims 7-9,
ii) obtaining transformed plant cells,
iii) regenerating from said transformed plant cells genetically transformed plants, and
iv) optionally, propagating said plants.

23. Process according to claim 22 wherein said pathogen is a nematode, and preferably a root-knot nematode.

24. Process according to claim 22 or 23 wherein said nematode is *Meloigogyne incognita*.

25. Process for protecting plants in cultivation against pathogen infection, which comprises:
i) providing the genome of plants with a DNA construct according to any of claims 7-9, and
ii) growing said plants.

26. Process for isolating a nucleic acid according to claim 1-6, comprising the following steps:
i) screening a genomic or cDNA library of a plant with a DNA sequence according to claim 1-6,
ii) identifying positive clones which hybridize to said DNA sequence,
iii) isolating said positive clones.

27. The process of claim 26 wherein said library originates from a first plant and the DNA sequence belongs to a second plant.

28. Process of selective restriction fragment amplification for identifying a nucleic acid according to claim 1-6 using primer combinations identifying at least one of the AFLP markers PM02 to PM29 as depicted in Table 3.

29. The process of claim 28 wherein said primer combination identifies AFLP marker PM14.

30. An oligonucleotide comprising a DNA sequence which corresponds to at least part of the nucleic acid according to claim 1-6.

31. The oligonucleotide of claim 30, which is of a size sufficient to hybridize selectively to the DNA sequence of any of claims 1 to 6 under stringent hybridization conditions.

32. An oligonucleotide according to claim 31 wherein said DNA sequence corresponds to the sequence starting at nucleotide 6921 and ending at nucleotide 7034.

33. An oligonucleotide according to claim 32 wherein said DNA sequence is located at the 3'end, and preferably corresponds to the sequence 5'-TGCAGGA-3', which can prime the synthesis of DNA.

34. An oligonucleotide according to claim 32 wherein said DNA sequence is located at the 3'end, and preferably corresponds to the sequence 5'-TAATCT-3' which can prime the synthesis of DNA.

35. A primer combination comprising a first oligonucleotide according to claim 33 and a second oligonucleotide according to claim 34.

36. Diagnostic kit comprising at least one oligonucleotide according to any of claims 30-34.

37. Diagnostic kit comprising a primer combination according to claim 35.

38. Process for detecting the presence or absence of a DNA sequence according to claim 1-6, particularly in a plant DNA using a diagnostic kit according to claim 36 or 37.

39. A polypeptide having an amino acid sequence having the sequence provided in figure 5 or coded by the corresponding homologous sequence according to claim 1 or 2.

40. A RNA having a ribonucleic acid sequence of a transcript of part or all of the DNA sequence of claim 1 or 2.


SEQUENCE LISTING

(1) GENERAL INFORMATION:

(i) APPLICANT:
(A) NAME: KEYGENE N.V.
(B) STREET: Agrobusiness Park 90
(C) CITY: WAGENINGEN
(E) COUNTRY: THE NETHERLANDS
(F) POSTAL CODE (ZIP): 6700 AE

(ii) TITLE OF INVENTION: RESISTANCE AGAINST NEMATODES

(iii) NUMBER OF SEQUENCES: 15

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 96401764.4

(2) INFORMATION FOR SEQ ID NO: 1:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "OLIGONUCLEOTIDE"

(xi) SEQUENCE DESCRIPTION: SEQ ID NO: 1:

GACTGCGTAC ATGCAGNN 18

(2) INFORMATION FOR SEQ ID NO: 2:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "OLIGONUCLEOTIDE"

(xi) SEQUENCE DESCRIPTION: SEQ ID NO: 2:

GATGAGTCCT GAGTAANNN 19

(2) INFORMATION FOR SEQ ID NO: 3:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "OLIGONUCLEOTIDE"

(ix) FEATURE:
(A) NAME/KEY: misc_feature
(B) LOCATION:4..17
(D) OTHER INFORMATION:/note= "SEQUENCE COMPLEMENTARY TO
SEQ ID NO: 4"

(ix) FEATURE:
(A) NAME/KEY: misc_feature
(B) LOCATION:1..21
(D) OTHER INFORMATION:/note= "PstI-adapter"

(xi) SEQUENCE DESCRIPTION: SEQ ID NO: 3:

CTCGTAGACT GCGTACATGC A 21

(2) INFORMATION FOR SEQ ID NO: 4:

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "OLIGONUCLEOTIDE"

(ix) FEATURE:
(A) NAME/KEY: misc_feature
(B) LOCATION:1..14
(D) OTHER INFORMATION:/note= "SEQUENCE COMPLEMENTARY TO
SEQ ID NO: 3"

(xi) SEQUENCE DESCRIPTION: SEQ ID NO: 4:

TGTACGCAGT CTAC 14

(2) INFORMATION FOR SEQ ID NO: 5:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "OLIGONUCLEOTIDE"

(ix) FEATURE:
(A) NAME/KEY: misc_feature
(B) LOCATION:1..16
(D) OTHER INFORMATION:/note= "MseI-adapter"

(xi) SEQUENCE DESCRIPTION: SEQ ID NO: 5:

GACGATGAGT CCTGAG 16

(2) INFORMATION FOR SEQ ID NO: 6:

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "OLIGONUCLEOTIDE"

(ix) FEATURE:
(A) NAME/KEY: misc_feature
(B) LOCATION:1..14
(D) OTHER INFORMATION:/note= "SEQUENCE COMPLEMENTARY TO
SEQ ID NO:5"

(xi) SEQUENCE DESCRIPTION: SEQ ID NO: 6:

TACTCAGGAC TCAT 14

(2) INFORMATION FOR SEQ ID NO: 7:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "OLIGONUCLEOTIDE"

(ix) FEATURE:
(A) NAME/KEY: misc_feature
(B) LOCATION:1..18
(D) OTHER INFORMATION:/note= "PstI-primers"

(xi) SEQUENCE DESCRIPTION: SEQ ID NO: 7:

GACTGCGTAC ATGCAGNN 18

(2) INFORMATION FOR SEQ ID NO: 8:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "OLIGONUCLEOTIDE"

(ix) FEATURE:

(A) NAME/KEY: misc_feature
(B) LOCATION:1..19
(D) OTHER INFORMATION:/note= "MseI-primers"

(xi) SEQUENCE DESCRIPTION: SEQ ID NO: 8:

GATGAGTCCT GAGTAANNN 19

(2) INFORMATION FOR SEQ ID NO: 9:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "OLIGONUCLEOTIDE"

(xi) SEQUENCE DESCRIPTION: SEQ ID NO: 9:

GACTGCGTAC ATGCAG 16

(2) INFORMATION FOR SEQ ID NO: 10:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "OLIGONUCLEOTIDE"

(xi) SEQUENCE DESCRIPTION: SEQ ID NO: 10:

GATGAGTCCT GAGTAA 16

(2) INFORMATION FOR SEQ ID NO: 11:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "OLIGONUCLEOTIDE"

(xi) SEQUENCE DESCRIPTION: SEQ ID NO: 11:

GACTGCGTAC ATGCAGGA 18

(2) INFORMATION FOR SEQ ID NO: 12:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "OLIGONUCLEOTIDE"

(xi) SEQUENCE DESCRIPTION: SEQ ID NO: 12:

GATGAGTCCT GAGTAATCT 19

(2) INFORMATION FOR SEQ ID NO: 13:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "OLIGONUCLEOTIDE"

(xi) SEQUENCE DESCRIPTION: SEQ ID NO: 13:

GACTGCGTAC CAATTC 16

(2) INFORMATION FOR SEQ ID NO: 14:

(i) SEQUENCE CHARACTERISTICS:
(A) LENGTH: 9870 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: CDS
(B) LOCATION:3491..7112

(xi) SEQUENCE DESCRIPTION: SEQ ID NO: 14:

TTTTCCTCTT CATATAACTT TTTCCTTAAC CCCTCTCATG AATAATATAA TTGATGTGGA 60

TAAAGTATTA TCCTTTATGA TAAATAACGA AATTTAATAA TTTAAAGGGT GCAAATCTAT 120

AAAATGGAGA CGCACATTGA TAATGTCCTC TTGATTATTA TTAAAGAATT ACTCTAGCTT 180

CACAAATTTA AATTCATTAA TGCTTAATTA CATGATAAAA ACTTTAGTTG TTCTTTTTAC 240

ATGGTTTGCT AACTTTAATT TTTTTTCTTC ATATTCTTCA TTTGTTTATT ATTATTTTCT 300

AATTACTTAT TTAACTTTTA TACTCTTAAT ATTCATAACT CTCATCTTTT CATATTCATA 360

ACCTCCAAAT ATTTAAACTA AAACTTTAAG ATATCTTTTG ATATTTGTTC AATAATAAAT 420
TCAACTTCTT TATCTTATGA AACCCCTACC AAGATTATTA GGCTATTATT TTTTATTCTA 480
TAGTAAAAAC AAATGATGAA GATTCTTGAA TTTTATAGGA TATGAAAGAA GTCGATAAAA 540
TCTCAGAGAG TTATGTACTA ATTTTGTACT TATTTTTTCA TCTATATATA CATAAATCTT 600
ATAAGAATAA TGTCTATATT GTATTTTTTT CTTAAATATT ATGTTTCTTT TTAATTTTTT 660
TTCACTCTGT TAGACTTCTT AATTTAGTTT TCTATGAATG TTTTATTGCC GTAAGTCTTT 720
GAATTTTGTA ATTGTTACAT TTTATTATTC ATTACGATTT ACATATATAT TTCCATGAGA 780
TTTGGTCATT CTAACGTATC TATAAAAATT CACATGAAAC ACACGTGTGA AGCGCATCCT 840
CAGAAAAACT AGTGTATATA TATATATATA TATATATATA TATATATATA TATATATATA 900
TATATATATA TATATATATA TATATATTAT TCTTATTAAA AAAGAATGTC CTTATTTCAT 960
TTTTAATCTG GTTAAAAAAG AATAATCTCT TTCCTTTTTT GACAATATTT TAACTTTAAC 1020
TTTCCACGTA ACATGTTTAA GACAACAAAA TTAAATGACA TTTTAATCTT GTAACATAGA 1080
AAAGTAACAT ATGATAATTG TCGTTGTCCC TAAACATGAT AGATGTATAA TTCAAAAGTC 1140
AATGAATTGT ATTTTAGTAT TATATTATGA ATGAACAAAC TGTCAAGATG TGTATATATA 1200
TATATATTTT ATTCTTGTTA ATTTGGCCTT TCAAGTAATT AATTCATTGT TAGGCAGTTG 1260
AATTAATAAT CTCTTTTAGG AATCTTCCCA TGTGAATAAC AAGACTTATA ATAATAATAA 1320
TAAAGTCCAG ATCTTGTTTC AATTGGATCA TTTGGCAAAC AATTACTCTG TTTCTGAAAC 1380
AAGGAATAGG GCTTCTAATA TTGTAGGGGA TTTTTTTTTC TTCATTAATT TATACTTATG 1440
ATATTAATTA TTGTTTTTGA GTACATATTT TAAACTCTGT TGTTTATTTT TCTGCAAAGT 1500
TTCTCCGGTT ATATTGAACA TATACACATA TAGTACATAT ATTTATTGTA AAAAAAATAA 1560
TTATTATACT CCATTTCAAG AAATTATGTT TTGATATTAT ATATTAAATT CTATAATGTG 1620
GAAATTGTCA ATGTCTACAA TGTGTTTGAT GAAATGACAA CCACTTGTTT TTATCTGCAA 1680
CAGTATAAAA ATTGGCTTTG CTTCTTTTAG ATTAATATAA TATTTTACAG GTCACATATT 1740
ATATTTATAT TGTGAAAGAC AAGAGATATT GATTAAAAAA AGACTTATGG GTTTGTATTT 1800
TAATATTTCA TTCTTCTTCA TTACTAAAAG ACTTGTATCG TATATTTCAA CTACTACACT 1860
TGTTTTCTTA TCCAATAGCT TCAACATTAT TTCTCAAACA AAGGGTTCTC TAGCTAAACT 1920
TCAGCCTGTG TAAAGGTAAC ATCTTCTTTA TTCACAGCAT AATAACAATG AATTTGGTCG 1980
ATGTTTGAAG TAAGCTTGAA ATTTTCTCTT TCTAAGTTTG TTTGATCCAT TTAGATTCTT 2040
TTAAATACTT TTGGTATTTA AAGGACTTGT GAAGTCAATG AATTGTATTT TAGTAATCTT 2100
GCAATTCTAG ATCTAGCTAT TTGTTGTTCT CCTTTCAACC AAACTACTTC TTCAATTTGT 2160
CTAACAAAAA TATGTCAAAA AGGTATGAAC ATGCTTAATC GGAGATCTTT ATTGATTCTA 2220
CTTCAGCTAC TCTAAAAAAA AATCTTTTTT CCATTAAGCC CAAGTCGAGA TAGGAGAAAA 2280

ATATTATTAG AGAGATTATT AATTTAATGA CATTTTACTC TAGTTTTTTA TCAAAATAAG 2340

GGAATAATAT CCTGTTATTT AACTACCTTT TAAGCATTAT GGGTGGAAAG TAGAAAGAAG 2400

AAACATAACA GAACAGACAG TAAGTTATGC TTTAATGAGT AGATCTGTAT AGGATTACAT 2460

ATTTGTTTGA CTTTTCGGTG TTTCGATTAG AAAACTTACA AGTTTTTAAT ACATGTATCA 2520

TTTGTTGATT TGTCCGTTTG GCACGTCATC TGTGGTTACA AGTCACATAT GAAGTATGTC 2580

CACGAGACAC ACCGAATGTC AAGTATAGAT TTCTACTTGA TCATACACAA CTTTATCTGA 2640

GGTTGATGCC AAATTTAAAT GACTACCTAA AGCTGATATT TTAAACATTA ATCTTGTACA 2700

CGAAAACATT ATTCCTATTA CTGTTTTCTT TACCTTTACC TTATAGACTT TTTTGGCAGA 2760

AAAAAGTTAG ACAGATACAT TTGATGATGT TTACCATTCT CATTCTCTCT TTATTTTATT 2820

TTCTTTACAT TCACACGCAC AATAATTTTC TTGTAGGTTC CTTATATGCC ATATGCACAT 2880

AGACGAATCT AGGATTTGAT ATTTACAAGT TTCTATGTCG ACGTCATATT AATATCAATA 2940

ATAATTAGAT TGACAATCAC ATATTTATAA TATTAAGTCG ATAACTTTCT TCTTTGTATA 3000

GGTTGGAAAA GTAATGGTAA ACGAGCAGGA CTCCTTTTTC TTTTTTTTGT AAATAATTAA 3060

CAGTTGTGAG ATTTTATGTT TGTGACTTCA TGTCATAAAC ATTTTGATGT GTGATTAAGA 3120

TTGACATTTC CAATTGTGCG AGTCTAAAAT TACTATATGT GAAAATAGTG ATATTATTGA 3180

TTATTCGTAT TTTTTCATCT TCTTTCTCCT GTTAAAGTTT TATCTACTTT TTATTCATCA 3240

GGTCTTGAGA AAAAGTAGAA TCATGGAAAA ACGAAAAGAT ATTGAAGAAG CAAACAACTC 3300

ATTGGTATGT TATTTTATAG AGTAAACTGT AAAGTATTGA ATTATAGATA TGTGGCTTTA 3360

AAATGTATTA TTTTGGCAGG TGTTATTTTC TGCTCTTAGC AAGGACATTG CCAATGTTCT 3420

AATTTTCCTA GAGAATGAGG AAAATCAAAA AGCTCTTGAC AAAGATCAAG TTGAAAAGCT 3480

AAAATTGAAA ATG GCA TTT ATT TGT ACA TAT GTT CAG CTT TCT TAT TCC 3529
Met Ala Phe Ile Cys Thr Tyr Val Gln Leu Ser Tyr Ser
1               5                   10

GAT TTT GAG CAG TTT GAA GAT ATA ATG ACT AGA AAT AGA CAA GAG GTT 3577
Asp Phe Glu Gln Phe Glu Asp Ile Met Thr Arg Asn Arg Gln Glu Val
    15                  20                  25

GAG AAT CTG CTT CAA TCA CTT TTG GAT GAT GAT GTC CTT ACT AGC CTC 3625
Glu Asn Leu Leu Gln Ser Leu Leu Asp Asp Asp Val Leu Thr Ser Leu
30                  35                  40                  45

ACC AGT AAT ATG GAT GAC TGT ATC AGC TTG TAT CAT CGT TCT TAT AAA 3673
Thr Ser Asn Met Asp Asp Cys Ile Ser Leu Tyr His Arg Ser Tyr Lys
                50                  55                  60

TCA GAT GCC ATC ATG ATG GAT GAG CAA TTG GAC TTC CTC CTC TTG AAT 3721
Ser Asp Ala Ile Met Met Asp Glu Gln Leu Asp Phe Leu Leu Leu Asn
            65                  70                  75

CTG TAT CAT CTA TCC AAG CAT CAC GCT GAA AAG ATA TTT CCT GGA GTG 3769
Leu Tyr His Leu Ser Lys His His Ala Glu Lys Ile Phe Pro Gly Val
        80                  85                  90

ACT CAA TAT GAA GTT CTT CAG AAT GTA TGT GGC AAC ATA AGA GAT TTC 3817
Thr Gln Tyr Glu Val Leu Gln Asn Val Cys Gly Asn Ile Arg Asp Phe
    95                  100                 105

CAT GGG TTG ATA CTG AAT GGT TGC ATT AAG CAT GAG ATG GTT GAG AAT 3865
His Gly Leu Ile Leu Asn Gly Cys Ile Lys His Glu Met Val Glu Asn
110                 115                 120                 125

GTC TTA CCT CTG TTT CAA CTC ATG GCT GAA AGA GTA GGA CAC TTC CTT 3913
Val Leu Pro Leu Phe Gln Leu Met Ala Glu Arg Val Gly His Phe Leu
                130                 135                 140

TGG GAG GAT CAG ACT GAT GAA GAC TCT CGG CTC TCC GAG CTA GAT GAG 3961
Trp Glu Asp Gln Thr Asp Glu Asp Ser Arg Leu Ser Glu Leu Asp Glu
            145                 150                 155

GAT GAA CAC AAT GAT AGA GAC TCT CGA CTC TTC CAG CTA ACA CAT CTA 4009
Asp Glu His Asn Asp Arg Asp Ser Arg Leu Phe Gln Leu Thr His Leu
        160                 165                 170

CTC TTG AAG ATT GTT CCA ACT GAA CTG GAG GTT ATG CAC ATA TGT TAT 4057
Leu Leu Lys Ile Val Pro Thr Glu Leu Glu Val Met His Ile Cys Tyr
    175                 180                 185

ACA AAT TTG AAA GCT TCA ACT TCA GCA GAA GTT GGA CGC TTC ATT AAG 4105
Thr Asn Leu Lys Ala Ser Thr Ser Ala Glu Val Gly Arg Phe Ile Lys
190                 195                 200                 205

AAG CTC CTG GAA ACC TCA CCG GAT ATT CTC AGA GAA TAT ATC ATT CAA 4153
Lys Leu Leu Glu Thr Ser Pro Asp Ile Leu Arg Glu Tyr Ile Ile Gln
                210                 215                 220

CTA CAA GAG CAT ATG TTA ACT GTT ATT CCC CCT AGC ACT TTA GGG GCT 4201
Leu Gln Glu His Met Leu Thr Val Ile Pro Pro Ser Thr Leu Gly Ala
            225                 230                 235

CGA AAC ATT CAT GTC ATG ATG GAA TTC CTA TTA CTT ATT CTT TCT GAT 4249
Arg Asn Ile His Val Met Met Glu Phe Leu Leu Leu Ile Leu Ser Asp
        240                 245                 250

ATG CCC AAG GAC TTT ATT CAT CAT GAC AAA CTT TTT GAT CTC TTG GCT 4297
Met Pro Lys Asp Phe Ile His His Asp Lys Leu Phe Asp Leu Leu Ala
    255                 260                 265

CAT GTT GGA ACA CTT ACC AGG GAG GTA TCG ACT CTT GTA CGT GAC TTG 4345
His Val Gly Thr Leu Thr Arg Glu Val Ser Thr Leu Val Arg Asp Leu
270                 275                 280                 285

GAA GAG AAA TTA AGG AAT AAA GAG GGT AAT AAC CAA ACA AAT TGT GCA 4393
Glu Glu Lys Leu Arg Asn Lys Glu Gly Asn Asn Gln Thr Asn Cys Ala
                290                 295                 300

ACC CTA GAC TTG CTG GAA AAT ATT GAA CTC CTC AAG AAA GAT CTC AAA 4441
Thr Leu Asp Leu Leu Glu Asn Ile Glu Leu Leu Lys Lys Asp Leu Lys
            305                 310                 315

CAT GTT TAT CTG AAA GCC CCA AAT TCA TCT CAA TGT TGC TTC CCC ATG 4489
His Val Tyr Leu Lys Ala Pro Asn Ser Ser Gln Cys Cys Phe Pro Met
        320                 325                 330

AGT GAT GGA CCA CTC TTC ATG CAT CTT CTA CAC ATG CAC TTA AAT GAT 4537
Ser Asp Gly Pro Leu Phe Met His Leu Leu His Met His Leu Asn Asp
    335                 340                 345

TTG CTA GAT TCT AAT GCT TAT TCA ATT TCT TTG ATA AAG GAA GAA ATC 4585
Leu Leu Asp Ser Asn Ala Tyr Ser Ile Ser Leu Ile Lys Glu Glu Ile
350                 355                 360                 365

GAG TTG GTG AGT CAA GAA CTG GAA TTC ATA AGA TCA TTC TTT GGG GAT 4633
Glu Leu Val Ser Gln Glu Leu Glu Phe Ile Arg Ser Phe Phe Gly Asp
                370                 375                 380

GCT GCT GAG CAA GGA TTG TAT AAA GAT ATC TGG GCA CGT GTT CTA GAT 4681
Ala Ala Glu Gln Gly Leu Tyr Lys Asp Ile Trp Ala Arg Val Leu Asp
            385                 390                 395

GTG GCT TAT GAG GCA AAA GAT GTC ATA GAT TCA ATT ATT GTT CGA GAT 4729
Val Ala Tyr Glu Ala Lys Asp Val Ile Asp Ser Ile Ile Val Arg Asp
        400                 405                 410

AAT GGT CTC TTA CAT CTT ATT TTC TCA CTT CCC ATT ACC ATA AAG AAG 4777
Asn Gly Leu Leu His Leu Ile Phe Ser Leu Pro Ile Thr Ile Lys Lys
    415                 420                 425

ATC AAA CTT ATC AAA GAA GAG ATC TCT GCT TTA GAT GAG AAC ATT CCC 4825
Ile Lys Leu Ile Lys Glu Glu Ile Ser Ala Leu Asp Glu Asn Ile Pro
430                 435                 440                 445

AAG GAC AGA GGT CTA ATC GTT GTG AAC TCT CCC AAG AAA CCA GTT GAG 4873
Lys Asp Arg Gly Leu Ile Val Val Asn Ser Pro Lys Lys Pro Val Glu
                450                 455                 460

AGA AAG TCA TTG ACA ACT GAT AAA ATA ATT GTA GGT TTT GAG GAG GAG 4921
Arg Lys Ser Leu Thr Thr Asp Lys Ile Ile Val Gly Phe Glu Glu Glu
            465                 470                 475

ACA AAC TTG ATA CTT AGA AAG CTC ACC AGT GGA CCC GCA GAT TTA GAT 4969
Thr Asn Leu Ile Leu Arg Lys Leu Thr Ser Gly Pro Ala Asp Leu Asp
        480                 485                 490

GTC ATT TCG ATC ACC GGT ATG CCG GGT TCA GGT AAA ACT ACT TTG GCA 5017
Val Ile Ser Ile Thr Gly Met Pro Gly Ser Gly Lys Thr Thr Leu Ala
    495                 500                 505

TAC AAA GTA TAC AAT GAT AAG TCA GTT TCT AGA CAT TTT GAC CTT CGT 5065
Tyr Lys Val Tyr Asn Asp Lys Ser Val Ser Arg His Phe Asp Leu Arg
510                 515                 520                 525

GCA TGG TGC ACG GTC GAT CAA GGA TAT GAC GAC AAG AAG TTG TTG GAT 5113
Ala Trp Cys Thr Val Asp Gln Gly Tyr Asp Asp Lys Lys Leu Leu Asp
                530                 535                 540

ACA ATT TTC AGT CAA GTT AGT GGC TCA GAT TCA AAT TTG AGT GAG AAT 5161
Thr Ile Phe Ser Gln Val Ser Gly Ser Asp Ser Asn Leu Ser Glu Asn
            545                 550                 555

ATT GAT GTT GCT GAT AAA TTG CGG AAA CAA CTG TTT GGA AAG AGG TAT 5209
Ile Asp Val Ala Asp Lys Leu Arg Lys Gln Leu Phe Gly Lys Arg Tyr
        560                 565                 570

CTT ATT GTC TTA GAT GAT GTG TGG GAT ACT ACT ACA TTG GAT GAG TTG 5257
Leu Ile Val Leu Asp Asp Val Trp Asp Thr Thr Thr Leu Asp Glu Leu
    575                 580                 585

ACA AGA CCT TTT CCT GAA GCT AAG AAA GGA AGT AGG ATT ATT TTG ACA 5305
Thr Arg Pro Phe Pro Glu Ala Lys Lys Gly Ser Arg Ile Ile Leu Thr
590                 595                 600                 605

ACT CGA GAA AAG GAA GTG GCT TTG CAT GGA AAG CTG AAC ACT GAT CCT 5353
Thr Arg Glu Lys Glu Val Ala Leu His Gly Lys Leu Asn Thr Asp Pro
                610                 615                 620

CTT GAC CTT CGA TTG CTA AGA CCA GAT GAA AGT TGG GAA CTT TTA GAG 5401
Leu Asp Leu Arg Leu Leu Arg Pro Asp Glu Ser Trp Glu Leu Leu Glu
            625                 630                 635

AAA AGG ACA TTT GGT AAT GAG AGT TGC CCT GAT GAA CTA TTA GAT GTC 5449
Lys Arg Thr Phe Gly Asn Glu Ser Cys Pro Asp Glu Leu Leu Asp Val
        640                 645                 650

GGT AAA GAA ATA GCC GAA AAT TGT AAA GGG CTT CCT TTG GTG GCT GAT 5497
Gly Lys Glu Ile Ala Glu Asn Cys Lys Gly Leu Pro Leu Val Ala Asp
    655                 660                 665

CTG ATT GCT GGA GTC ATT GCT GGG AGG GAA AAG AAA AGG AGT GTG TGG 5545
Leu Ile Ala Gly Val Ile Ala Gly Arg Glu Lys Lys Arg Ser Val Trp
670                 675                 680                 685

CTT GAA GTT CAA AGT AGT TTG AGT TCT TTT ATT TTG AAC AGT GAA GTG 5593
Leu Glu Val Gln Ser Ser Leu Ser Ser Phe Ile Leu Asn Ser Glu Val
                690                 695                 700

GAA GTG ATG AAA GTT ATA GAA TTA AGT TAT GAC CAT TTA CCA CAT CAC 5641
Glu Val Met Lys Val Ile Glu Leu Ser Tyr Asp His Leu Pro His His
            705                 710                 715

CTC AAG CCA TGC TTG CTT CAC TTT GCA AGT TGG CCG AAG GAC ACT CCT 5689
Leu Lys Pro Cys Leu Leu His Phe Ala Ser Trp Pro Lys Asp Thr Pro
        720                 725                 730

TTG ACA ATC TAT TTG TTG ACT GTT TAT TTG GGT GCT GAA GGA TTT GTG 5737
Leu Thr Ile Tyr Leu Leu Thr Val Tyr Leu Gly Ala Glu Gly Phe Val
    735                 740                 745

GAA AAG ACG GAG ATG AAG GGT ATA GAA GAA GTG GTG AAG ATT TAT ATG 5785
Glu Lys Thr Glu Met Lys Gly Ile Glu Glu Val Val Lys Ile Tyr Met
750                 755                 760                 765

GAT GAT TTA ATT TCC AGT AGC TTG GTA ATT TGT TTC AAT GAG ATA GGT 5833
Asp Asp Leu Ile Ser Ser Ser Leu Val Ile Cys Phe Asn Glu Ile Gly
                770                 775                 780

GAT ATA CTG AAT TTC CAA ATT CAT GAT CTT GTG CAT GAC TTT TGT TTG 5881
Asp Ile Leu Asn Phe Gln Ile His Asp Leu Val His Asp Phe Cys Leu
            785                 790                 795

ATA AAA GCA AGA AAG GAA AAT TTG TTT GAT CGG ATA AGA TCA AGT GCT 5929
Ile Lys Ala Arg Lys Glu Asn Leu Phe Asp Arg Ile Arg Ser Ser Ala
        800                 805                 810

CCA TCA GAT TTG TTG CCT CGT CAA ATT ACC ATT GAT TAT GAT GAG GAG 5977
Pro Ser Asp Leu Leu Pro Arg Gln Ile Thr Ile Asp Tyr Asp Glu Glu
    815                 820                 825

GAG GAG CAC TTT GGG CTT AAT TTT GTC ATG TTC GAT TCA AAT AAG AAA 6025
Glu Glu His Phe Gly Leu Asn Phe Val Met Phe Asp Ser Asn Lys Lys
830                 835                 840                 845

AGG CAT TCT GGT AAA CAC CTC TAT TCT TTG AGG ATA AAT GGA GAC CAG 6073
Arg His Ser Gly Lys His Leu Tyr Ser Leu Arg Ile Asn Gly Asp Gln
                850                 855                 860

CTG GAT GAC AGT GTT TCT GAT GCA TTT CAC CTA AGA CAC TTG AGG CTT 6121
Leu Asp Asp Ser Val Ser Asp Ala Phe His Leu Arg His Leu Arg Leu
            865                 870                 875

ATT AGA GTG TTG GAC CTG GAA CCC TCT TTA ATC ATG GTG AAT GAT TCT 6169
Ile Arg Val Leu Asp Leu Glu Pro Ser Leu Ile Met Val Asn Asp Ser
        880                 885                 890

TTG CTG AAT GAA ATA TGC ATG TTG AAT CAT TTG AGG TAC TTA AGA ATT 6217
Leu Leu Asn Glu Ile Cys Met Leu Asn His Leu Arg Tyr Leu Arg Ile
    895                 900                 905

CGG ACA CAA GTT AAA TAT CTG CCT TTC TCT TTC TCA AAC CTC TGG AAT 6265
Arg Thr Gln Val Lys Tyr Leu Pro Phe Ser Phe Ser Asn Leu Trp Asn
910                 915                 920                 925

CTA GAA AGT CTG TTT GTG TCT AAC AAA GGA TCA ATC TTG GTA CTA TTA 6313
Leu Glu Ser Leu Phe Val Ser Asn Lys Gly Ser Ile Leu Val Leu Leu
                930                 935                 940

CCG AGA ATT TTG GAT CTT GTA AAG TTG CGA GTG CTG TCC GTG GGT GCT 6361
Pro Arg Ile Leu Asp Leu Val Lys Leu Arg Val Leu Ser Val Gly Ala
            945                 950                 955

TGT TCT TTC TTT GAT ATG GAT GCA GAT GAA TCA ATA TTG ATA GCA AAG 6409
Cys Ser Phe Phe Asp Met Asp Ala Asp Glu Ser Ile Leu Ile Ala Lys
        960                 965                 970

GAC ACA AAG TTA GAG AAC TTG AGA ATA TTA GGG GAA CTG TTG ATT TCC 6457
Asp Thr Lys Leu Glu Asn Leu Arg Ile Leu Gly Glu Leu Leu Ile Ser
    975                 980                 985

TAT TCG AAA GAT ACA ATG AAT ATT TTC AAA AGG TTT CCC AAT CTT CAG 6505
Tyr Ser Lys Asp Thr Met Asn Ile Phe Lys Arg Phe Pro Asn Leu Gln
990                 995                 1000                1005

GTG CTT CAG TTT GAA CTC AAG GAG TCA TGG GAT TAT TCA ACA GAG CAA 6553
Val Leu Gln Phe Glu Leu Lys Glu Ser Trp Asp Tyr Ser Thr Glu Gln
                1010                1015                1020

CAT TGG TTC CCG AAA TTG GAT TGC CTA ACT GAA CTA GAA ACA CTC TGT 6601
His Trp Phe Pro Lys Leu Asp Cys Leu Thr Glu Leu Glu Thr Leu Cys
            1025                1030                1035

GTA GGT TTT AAA AGT TCA AAC ACA AAC CAC TGT GGG TCC TCT GTT GCG 6649
Val Gly Phe Lys Ser Ser Asn Thr Asn His Cys Gly Ser Ser Val Ala
        1040                1045                1050

ACA AAT CGG CCG TGG GAT TTT CAC TTC CCT TCA AAT TTG AAA GAA CTG 6697
Thr Asn Arg Pro Trp Asp Phe His Phe Pro Ser Asn Leu Lys Glu Leu
    1055                1060                1065

TTG TTG TAT GAC TTT CCT CTG ACA TCC GAT TCA CTA TCA ACA ATA GCG 6745
Leu Leu Tyr Asp Phe Pro Leu Thr Ser Asp Ser Leu Ser Thr Ile Ala
1070                1075                1080                1085

AGA CTG CCC AAC CTT GAA AAT TTG TCC CTT TAT GAT ACA ATC ATC CAG 6793
Arg Leu Pro Asn Leu Glu Asn Leu Ser Leu Tyr Asp Thr Ile Ile Gln
                1090                1095                1100

GGA GAA GAA TGG AAC ATG GGG GAG GAA GAC ACT TTT GAG AAT CTC AAA 6841
Gly Glu Glu Trp Asn Met Gly Glu Glu Asp Thr Phe Glu Asn Leu Lys
            1105                1110                1115

TTT TTG AAC TTG CGT CTA CTG ACT CTT TCC AAG TGG GAG GTT GGA GAG 6889
Phe Leu Asn Leu Arg Leu Leu Thr Leu Ser Lys Trp Glu Val Gly Glu
        1120                1125                1130

GAA TCC TTC CCC AAT CTT GAG AAA TTA AAA CTG CAG GAA TGT GGT AAG 6937
Glu Ser Phe Pro Asn Leu Glu Lys Leu Lys Leu Gln Glu Cys Gly Lys
    1135                1140                1145

CTT GAG GAG ATT CCA CCT AGT TTT GGA GAT ATT TAT TCA TTG AAA TTT 6985
Leu Glu Glu Ile Pro Pro Ser Phe Gly Asp Ile Tyr Ser Leu Lys Phe
1150                1155                1160                1165

ATC AAA ATT GTA AAG AGT CCT CAA CTT GAA GAT TCT GCT CTC AAG ATT 7033
Ile Lys Ile Val Lys Ser Pro Gln Leu Glu Asp Ser Ala Leu Lys Ile
                1170                1175                1180

AAG AAA TAC GCT GAA GAT ATG AGA GGA GGG AAC GAG CTT CAG ATC CTT 7081
Lys Lys Tyr Ala Glu Asp Met Arg Gly Gly Asn Glu Leu Gln Ile Leu
            1185                1190                1195

GGC CAG AAG AAT ATC CCC TTA TTT AAG TAG C ATTTTGGTTG AACTTTGCTT 7132
Gly Gln Lys Asn Ile Pro Leu Phe Lys *
        1200                1205

GGTGATATTG TATATGATTA AAATATCCTG TGATGAGATT CCTCTTAGTT TCTTTTAACA 7192

AAAAATATAA TTTTTATAAG TACACATATC GTTTGTTAAT TTGTCCATTT GTGATTGCAA 7252

GTCACACATG AGGTATGTTC GTATTATGGG TTTCAACTTG ATCAGACGTA ATTTTAAGAT 7312

AAGTGCTTAT ATGATGTTGC ATGCCAGATG GAAGTGACTA TGTGAAGTTT ATATTTTAAA 7372

CATTAATCTT GTATACCAAA CTACTATTCC TATGCTATGT TGTTTGCCAT TGTCGTTCTC 7432

TCTTTATTTT TTTTCTTTCC ATTCACACAC ACATTAATTT TCTAGTAGAC CGCATATTAC 7492

TACATCTGTA TTGTCCGTAT ACAAGACGAA TCCAGGATTT GATGTTTACA AGTATTTGTG 7552

AAGAATCCAG GATTTGATGT TTACAAGACA ATTAGATTCA TATATGTATA GGATTTTGAC 7612

AGAAACTGAG GGATTCACAT GACAATTACT CTGTGGATTT GCCTTTGGCT GTCCAAACCT 7672

CCTTTGTGTC TAACTTCGTC TGAAGTCCCA TTTATATGCT CAAAGCTCAG TCAAGGTACT 7732

GATTCAAAAG CTAGGCTGTG AAGTAAACTT TAAAATGATA TTGCTGCAAA GTCGCTCAAC 7792

AAAGGGTCAT AACCATCACT ACAACTACAC AAGCTCAAGC AAGTAAACGC GGGTGAAAGA 7852

TTAACATAGA TCGCTATCCC CTGCAAAAGC TAAGGAAAGC ATCTCTAACT TCTTAGCATG 7912

TACTCAAACA CACGATCTGT AAGGATGCCA GAAAGAGAAA GTTACGTTGC CGCAATTCCT 7972

TACAGTGTTG CACAATGTCC CCAAAACCAA CATCACACTA CAAAAAAAGG CTCAAATTCT 8032

GGGGGTTATA ATTAGACGGT CAATAACCCC TGCAATTTAG TGTTGTGGAG GTTGAATAAA 8092

CTCCTCCAAT TAGGAGTGTC ACAATTAAGT CGCGTGGGAT TCTTGGCACA TCCCGGTAAG 8152

GTTAACTAGC GGGGGTTTTG AACCCCAACC GCATTTCAAA CTAGGAGTCG AAACCCCAAC 8212

GATTTGTGAA CTCGGGGGAG TCAAAAACCC CCGCAATAAA TGATTTTTAC ATTAAAATTA 8272

ATAGGAGCTT GGACCCCTGT GATTTATGAA ATATAACTTT TTGTAGCATT TGCCAGAAAT 8332

ATTCAATTTT AGATACTAAT AATAAATTAA TTAACTAACA TGTGCATCAT TATTCAAAGG 8392

ACATATTAGT ATTAAGAAAT AATACAATAT TCAACACAAA AGTACCCAAA CTCAAGATAG 8452

GATCAGTTTA TGGAACTTCA ACTAGTTTCA CTATAATTAT TGTCACTAAC ATCAGCTGGC 8512

TGCAAAGGAG AATACATAAT AAGTGACTTT ATCCAAACTC AAAATCATGG CTGAATGTAG 8572

TAAAACACCA AAGATTATAA TAATTTCCAT TAATTATCAT ATACTACACA ACAACAAACT 8632

TAAAACAATA TAGAAAAGGA TTAAACCATT TACACAAGCA ATGATTCTAT ACCATTTCAA 8692

AACGACAACA TACTGTACTA CTAAACAAGA CACCATCAAA CTGATTTGGA CAAATATTAA 8752

CAATAGTTAA AACATGAACA AAGAATCTCA GGTTTCTTGT CAGTAGAAAA GAGACAGACT 8812

AGGAACTGGA GTGCTATTTT TCTTATAAGA GACAATTAAT GTTTACTTCT TTATATTTTG 8872

ACTATAAGTT GATTGGTTAT AATGTTTACG AGGTTGTATA TAATCCGATG TTCAATGATA 8932

TGACTTTCCT ATTGACTGAA ATGCTTGAAC GCAAACAGTA TATCTAGATT AAGAATGAGG 8992

ACGAATTACC TCTAGAGGCA TGGGTAATGG AAGCATAACT CCTTGATAAT GGTTGTTAGC 9052

CCACTGCAAG TCACAAAACA AAACATCCGT AATATTAACA TACTAAGGTT GTAAGCACTA 9112

AACGACAACA ACTATGCCTC AATCCCAACT AAGTTGGAAT CGACTATATG AATACTCACA 9172

ATTTCGATTT ATAGACAAAG ATACTAGTAG AAATGACGTC TTTCCTTTCT ATGTTAACAC 9232

TTGGACAGAG AATGTTAAAG ACTTACAACA ACAGAAAAGA GTTAAAATCA TTTAATTGAG 9292

CAAGGATTTC AAAACGACAA CACAATATAC TCAATTTTTC GACGGAAACA ACTGGTTGGA 9352

CAACAGTGCT ATTTGTAACT CCAATGAACA ACACTGCAAC GTACATGTAT CTCATTGCAC 9412

TAAATAAATC CCGTTGAGAG TAACATATCA ATAGTTACGA ACAATATGAT CACGACAAAG 9472

GATTGTAAGT ACCACAGGAC AAGTCATGCT TGCATGAAAA ACGGATATGT AAAGAACCAA 9532

AATCCTGCTG CTGAAATAAG CAGTTATGAT TATCCAAAAA TCATGAATAC ACATGCACTT 9592

GAGTTTGTTC CAAGAAAAAC ACAACCAACT ACTGTCGCAA GTGAAGATTC AAAAGTGACT 9652

ATTGATGTTA ATTCTTCCAC AAGGTTGAAT AATTTTGTCA CTATAGGATT TAAGACGAAG 9712

AAGAAACAGG CGACAATTTT GTAAGCATAG ACCTTCTTAT GCAACTATGA GCTGGTATGC 9772

TATTCATTTT CTTTACTCGT AAAAATCGTT GATACTAAAG AATGCCAATC CAGTCCTGCT 9832

GAATAGGCGC CAGGTGACTG GTTGCTGTTA ATAATTTT 9870

(2) INFORMATION FOR SEQ ID NO: 15:

(i) SEQUENCE CHARACTERISTICS:
(A) LENGTH: 1207 amino acids
(B) TYPE: amino acid
(D) TOPOLOGY: linear

(ii) MOLECULE TYPE: protein
(xi) SEQUENCE DESCRIPTION: SEQ ID NO: 15:

Met Ala Phe Ile Cys Thr Tyr Val Gln Leu Ser Tyr Ser Asp Phe Glu
1               5                   10                  15

Gln Phe Glu Asp Ile Met Thr Arg Asn Arg Gln Glu Val Glu Asn Leu
            20                  25                  30

Leu Gln Ser Leu Leu Asp Asp Asp Val Leu Thr Ser Leu Thr Ser Asn
        35                  40                  45

Met Asp Asp Cys Ile Ser Leu Tyr His Arg Ser Tyr Lys Ser Asp Ala
    50                  55                  60

Ile Met Met Asp Glu Gln Leu Asp Phe Leu Leu Leu Asn Leu Tyr His
65                  70                  75                  80

Leu Ser Lys His His Ala Glu Lys Ile Phe Pro Gly Val Thr Gln Tyr
                85                  90                  95

Glu Val Leu Gln Asn Val Cys Gly Asn Ile Arg Asp Phe His Gly Leu
            100                 105                 110

Ile Leu Asn Gly Cys Ile Lys His Glu Met Val Glu Asn Val Leu Pro
        115                 120                 125

Leu Phe Gln Leu Met Ala Glu Arg Val Gly His Phe Leu Trp Glu Asp
    130                 135                 140

Gln Thr Asp Glu Asp Ser Arg Leu Ser Glu Leu Asp Glu Asp Glu His
145                 150                 155                 160

Asn Asp Arg Asp Ser Arg Leu Phe Gln Leu Thr His Leu Leu Leu Lys
                165                 170                 175

Ile Val Pro Thr Glu Leu Glu Val Met His Ile Cys Tyr Thr Asn Leu
            180                 185                 190

Lys Ala Ser Thr Ser Ala Glu Val Gly Arg Phe Ile Lys Lys Leu Leu
        195                 200                 205

Glu Thr Ser Pro Asp Ile Leu Arg Glu Tyr Ile Ile Gln Leu Gln Glu
    210                 215                 220

His Met Leu Thr Val Ile Pro Pro Ser Thr Leu Gly Ala Arg Asn Ile
225                 230                 235                 240

His Val Met Met Glu Phe Leu Leu Leu Ile Leu Ser Asp Met Pro Lys
                245                 250                 255

Asp Phe Ile His His Asp Lys Leu Phe Asp Leu Leu Ala His Val Gly
            260                 265                 270

Thr Leu Thr Arg Glu Val Ser Thr Leu Val Arg Asp Leu Glu Glu Lys
        275                 280                 285

Leu Arg Asn Lys Glu Gly Asn Asn Gln Thr Asn Cys Ala Thr Leu Asp
    290                 295                 300

Leu Leu Glu Asn Ile Glu Leu Leu Lys Lys Asp Leu Lys His Val Tyr
305                 310                 315                 320

Leu Lys Ala Pro Asn Ser Ser Gln Cys Cys Phe Pro Met Ser Asp Gly
                325                 330                 335

Pro Leu Phe Met His Leu Leu His Met His Leu Asn Asp Leu Leu Asp
            340                 345                 350

Ser Asn Ala Tyr Ser Ile Ser Leu Ile Lys Glu Glu Ile Glu Leu Val
        355                 360                 365

Ser Gln Glu Leu Glu Phe Ile Arg Ser Phe Phe Gly Asp Ala Ala Glu
    370                 375                 380

Gln Gly Leu Tyr Lys Asp Ile Trp Ala Arg Val Leu Asp Val Ala Tyr
385                 390                 395                 400

Glu Ala Lys Asp Val Ile Asp Ser Ile Ile Val Arg Asp Asn Gly Leu
                405                 410                 415

Leu His Leu Ile Phe Ser Leu Pro Ile Thr Ile Lys Lys Ile Lys Leu
            420                 425                 430

Ile Lys Glu Glu Ile Ser Ala Leu Asp Glu Asn Ile Pro Lys Asp Arg
        435                 440                 445

Gly Leu Ile Val Val Asn Ser Pro Lys Lys Pro Val Glu Arg Lys Ser
    450                 455                 460

Leu Thr Thr Asp Lys Ile Ile Val Gly Phe Glu Glu Glu Thr Asn Leu
465                 470                 475                 480

Ile Leu Arg Lys Leu Thr Ser Gly Pro Ala Asp Leu Asp Val Ile Ser
                485                 490                 495

Ile Thr Gly Met Pro Gly Ser Gly Lys Thr Thr Leu Ala Tyr Lys Val
            500                 505                 510

Tyr Asn Asp Lys Ser Val Ser Arg His Phe Asp Leu Arg Ala Trp Cys
        515                 520                 525

Thr Val Asp Gln Gly Tyr Asp Asp Lys Lys Leu Leu Asp Thr Ile Phe
    530                 535                 540

Ser Gln Val Ser Gly Ser Asp Ser Asn Leu Ser Glu Asn Ile Asp Val
545                 550                 555                 560

Ala Asp Lys Leu Arg Lys Gln Leu Phe Gly Lys Arg Tyr Leu Ile Val
                565                 570                 575

Leu Asp Asp Val Trp Asp Thr Thr Thr Leu Asp Glu Leu Thr Arg Pro
            580                 585                 590

Phe Pro Glu Ala Lys Lys Gly Ser Arg Ile Ile Leu Thr Thr Arg Glu
        595                 600                 605

Lys Glu Val Ala Leu His Gly Lys Leu Asn Thr Asp Pro Leu Asp Leu
    610                 615                 620

Arg Leu Leu Arg Pro Asp Glu Ser Trp Glu Leu Leu Glu Lys Arg Thr
625                 630                 635                 640

Phe Gly Asn Glu Ser Cys Pro Asp Glu Leu Leu Asp Val Gly Lys Glu
                645                 650                 655

Ile Ala Glu Asn Cys Lys Gly Leu Pro Leu Val Ala Asp Leu Ile Ala
            660                 665                 670

Gly Val Ile Ala Gly Arg Glu Lys Lys Arg Ser Val Trp Leu Glu Val
        675                 680                 685

Gln Ser Ser Leu Ser Ser Phe Ile Leu Asn Ser Glu Val Glu Val Met
    690                 695                 700

Lys Val Ile Glu Leu Ser Tyr Asp His Leu Pro His His Leu Lys Pro
705                 710                 715                 720

Cys Leu Leu His Phe Ala Ser Trp Pro Lys Asp Thr Pro Leu Thr Ile
                725                 730                 735

Tyr Leu Leu Thr Val Tyr Leu Gly Ala Glu Gly Phe Val Glu Lys Thr
            740                 745                 750

Glu Met Lys Gly Ile Glu Glu Val Val Lys Ile Tyr Met Asp Asp Leu
        755                 760                 765

Ile Ser Ser Ser Leu Val Ile Cys Phe Asn Glu Ile Gly Asp Ile Leu
    770                 775                 780

Asn Phe Gln Ile His Asp Leu Val His Asp Phe Cys Leu Ile Lys Ala
785                 790                 795                 800

Arg Lys Glu Asn Leu Phe Asp Arg Ile Arg Ser Ser Ala Pro Ser Asp
                805                 810                 815

Leu Leu Pro Arg Gln Ile Thr Ile Asp Tyr Asp Glu Glu Glu Glu His
            820                 825                 830

Phe Gly Leu Asn Phe Val Met Phe Asp Ser Asn Lys Lys Arg His Ser
        835                 840                 845

Gly Lys His Leu Tyr Ser Leu Arg Ile Asn Gly Asp Gln Leu Asp Asp
    850                 855                 860

Ser Val Ser Asp Ala Phe His Leu Arg His Leu Arg Leu Ile Arg Val
865                 870                 875                 880

Leu Asp Leu Glu Pro Ser Leu Ile Met Val Asn Asp Ser Leu Leu Asn
                885                 890                 895

Glu Ile Cys Met Leu Asn His Leu Arg Tyr Leu Arg Ile Arg Thr Gln
            900                 905                 910

Val Lys Tyr Leu Pro Phe Ser Phe Ser Asn Leu Trp Asn Leu Glu Ser
        915                 920                 925

Leu Phe Val Ser Asn Lys Gly Ser Ile Leu Val Leu Leu Pro Arg Ile
    930                 935                 940

Leu Asp Leu Val Lys Leu Arg Val Leu Ser Val Gly Ala Cys Ser Phe
945                 950                 955                 960

Phe Asp Met Asp Ala Asp Glu Ser Ile Leu Ile Ala Lys Asp Thr Lys
                965                 970                 975

Leu Glu Asn Leu Arg Ile Leu Gly Glu Leu Leu Ile Ser Tyr Ser Lys
            980                 985                 990

Asp Thr Met Asn Ile Phe Lys Arg Phe Pro Asn Leu Gln Val Leu Gln
        995                 1000                1005

Phe Glu Leu Lys Glu Ser Trp Asp Tyr Ser Thr Glu Gln His Trp Phe
    1010                1015                1020

Pro Lys Leu Asp Cys Leu Thr Glu Leu Glu Thr Leu Cys Val Gly Phe
1025                1030                1035                1040

Lys Ser Ser Asn Thr Asn His Cys Gly Ser Ser Val Ala Thr Asn Arg
                1045                1050                1055

Pro Trp Asp Phe His Phe Pro Ser Asn Leu Lys Glu Leu Leu Leu Tyr
            1060                1065                1070

Asp Phe Pro Leu Thr Ser Asp Ser Leu Ser Thr Ile Ala Arg Leu Pro
        1075                1080                1085

Asn Leu Glu Asn Leu Ser Leu Tyr Asp Thr Ile Ile Gln Gly Glu Glu
    1090                1095                1100

Trp Asn Met Gly Glu Glu Asp Thr Phe Glu Asn Leu Lys Phe Leu Asn
1105                1110                1115                1120

Leu Arg Leu Leu Thr Leu Ser Lys Trp Glu Val Gly Glu Glu Ser Phe
                1125                1130                1135

Pro Asn Leu Glu Lys Leu Lys Leu Gln Glu Cys Gly Lys Leu Glu Glu
            1140                1145                1150

Ile Pro Pro Ser Phe Gly Asp Ile Tyr Ser Leu Lys Phe Ile Lys Ile
        1155                1160                1165

Val Lys Ser Pro Gln Leu Glu Asp Ser Ala Leu Lys Ile Lys Lys Tyr
    1170                1175                1180

Ala Glu Asp Met Arg Gly Gly Asn Glu Leu Gln Ile Leu Gly Gln Lys
1185                1190                1195                1200

Asn Ile Pro Leu Phe Lys  *
                1205